# EUROPEAN PATENT SPECIFICATION

(11) **EP 3 946 271 B1**
(45) Date of publication and mention of the grant of the patent: **25.12.2024**
(21) Application number: 20719561.1
(22) Date of filing: 02.04.2020
(51) Int. Cl.: A61K 9/16, A61K 47/36, A61K 31/00, A61K 31/05, A61K 31/12, A61K 31/192, A61K 31/352, A61K 31/19, A61K 31/37, A61K 31/353, A61K 31/40, A61K 31/4422, A61K 31/506, A61K 31/616, A61K 31/366

(54) **METHOD OF PRODUCTION OF A COMPOSITE OF YEAST-DERIVED BETA GLUCAN PARTICLE WITH INCORPORATED POORLY-WATER-SOLUBLE LOW-MOLECULAR-WEIGHT COMPOUND, PHARMACEUTICAL PREPARATION AND USE THEREOF**
VERFAHREN ZUR HERSTELLUNG EINES KOMPOSITS AUS VON HEFE-ABGELEITETEN BETA-GLUCAN-PARTIKELN MIT EINER EINGEBUNDENEN SCHWER WASSERLÖSLICHEN NIEDERMOLEKULAREN VERBINDUNG, PHARMAZEUTISCHE ZUBEREITUNG UND VERWENDUNG DAVON
PROCÉDÉ DE PRODUCTION D'UN COMPOSITE DE PARTICULE DE BÊTA-GLUCANE DÉRIVÉE DE LEVURE AVEC UN COMPOSÉ À FAIBLE POIDS MOLÉCULAIRE PEU SOLUBLE DANS L'EAU INCORPORÉ, PRÉPARATION PHARMACEUTIQUE ET UTILISATION DE CELUI-CI

(30) Priority: 04.04.2019 CZ 20192120
(43) Date of publication of application: 09.02.2022
(73) Proprietor: Vysoká skola chemicko-technologická v Praze, 16628 Praha 6 - Dejvice (CZ)
(72) Inventor: RUPHUY CHAN, Gabriela, 180 00 Praha 8 Liben (CZ); STEPANEK, Frantisek, 142 00 Praha 4 - Lhotka (CZ); HANUS, Jaroslav, 100 00 Praha 10 (CZ); SALAMUNOVA, Petra, 974 01 Banska Bystrica - Salkova (SK); SALON, Ivan, 065 31 Jarabina (SK)
(74) Representative: Hartvichova, Katerina
(86) International application number: PCT/CZ2020/050019
(87) International publication number: WO 2020/200337

(56) References cited:
- WO-A1-2005/113128
- CN-A- 105 749 290
- SALON IVAN ET AL: "Suspension stability and diffusion properties of yeast glucan microparticles", FOOD AND BIOPRODUCTS PROCESSING, INSTITUTION OF CHEMICAL ENGINEERS, RUGBY, GB, vol. 99, 6 May 2016 (2016-05-06), pages 128 - 135, XP029630944, ISSN: 0960-3085, DOI: 10.1016/J.FBP.2016.04.010
- SAMUELSEN A B ET AL: "Effects of orally administered yeast-derived beta-glucans: A review", MOLECULAR NUTRITION & FOOD RESEARCH, WILEY - VCH VERLAG, WEINHEIM, DE, vol. 58, no. 1, 1 January 2014 (2014-01-01), pages 183 - 193, XP002733985, ISSN: 1613-4125, [retrieved on 20130910], DOI: 10.1002/MNFR.201300338

## Description

### Field of Art

The present invention relates to a formulation of composites comprising yeast-derived beta glucan particles (GPs) and water-insoluble or poorly-water soluble compounds, such as medicaments (drugs) or food supplements. The composites can exhibit different crystallinity degrees depending on the formulation and, consequently, dissolution kinetics can be controlled. Yeast-derived beta-glucan particles are used as carriers for the encapsulation and amorphization of insoluble or poorly-water soluble compounds; amorphous formulations exhibiting faster dissolution rates, and consequently, enhanced oral bioavailability. The present invention further relates to a method of preparation of the composites by spray drying, and to the use thereof.

### Background Art

Poor solubility of active compounds, and their associated low dissolution rate in aqueous gastrointestinal fluids, is one of the most frequent causes of low bioavailability, mainly in the case of oral dosage forms, which are the most employed and convenient route of administration. Given that a vast majority of active compounds are insoluble/poorly soluble in water, efforts are focused in the development of strategies to improve the solubility of active compounds in water. Only in the pharmaceutical industry, about 90 % of drugs in the discovery pipeline and over 40 % with market approval are insoluble or poorly soluble in water.

One of the most promising methodologies for the improvement of solubility consists in the development of the compound as an amorphous solid. When compared to its crystalline counterpart, the amorphous form exhibits higher internal energy and, consequently, enhanced thermodynamic properties, including better solubility than the crystalline form thereof. However, the amorphous form of a compound is often unstable and tends to convert to a lower-energy crystalline state. The drawbacks of the current state of the art are thus low solubility of majority of compounds, such as medicaments (drugs) or food supplements, and their low stability and tendency to conversion into even less soluble crystalline form. One way of overcoming the above-mentioned drawbacks is incorporation of the compound in amorphous carriers, typically polymers.

The resulting composites are known as amorphous solid dispersions - a *dispersion* of the drug in an amorphous polymer matrix.

Glucan particles (GPs) are hollow and porous microspheres obtained from the cell wall of *Saccharomyces cerevisiae* (baker's yeast), mainly composed of polysaccharides (>85 % β-glucans). Since they are obtained from microorganisms, pattern recognition receptors of host immune cells can recognize them and trigger immune responses (Samuelsen, A.B.C., J. Schrezenmeir, and S.H. Knutsen, Effects of orally administered yeast-derived beta-glucans: A review. Molecular Nutrition & Food Research, 2014. 58(1): p. 183-193). For this reason, glucan particles have been of special interest as biotemplates for the encapsulation and macrophage-targeted delivery of drugs.

A wide range of water-soluble payloads, including peptides, siRNA, and DNA, have been encapsulated in glucan particles for targeted delivery. Moreover, studies of suspension stability and diffusion properties were done on water-soluble molecules with increasing molecular weights (caffeine, vitamin B12 and BSA) encapsulated in beta-glucan particles (Saloň I., et al. *Suspension stability and diffusion properties of yeast glucan microparticles.* Food and Bioproducts Processing, 2016. **99:** p. 128-135).

Regarding preparation methods, spray drying is a well-established technique that has been successfully used to encapsulate drugs or other compounds using water-soluble polymers. Typically, the drug and the polymeric carrier are both dissolved in water. This solution is then sprayed into a drying chamber, in which the solvent evaporates, producing composite particles that are further separated and collected. The encapsulation of water-soluble flavors in residual yeast cells by spray drying was reported by Sultana, A., et al., Microencapsulation of flavors by spray drying using Saccharomyces cerevisiae. Journal of Food Engineering, 2017. 199(Supplement C): p. 36-41. Available publications are focused on the use of spray drying as a final step in the preparation of yeast-derived beta glucan particles. Comparative studies have proven that, when compared to other drying methods, spray-drying leads to improved final properties of the GPs. The spray-dried GPs can be subsequently loaded with active compounds from aqueous solution; Upadhyay, T.K., et al., Preparation and characterization of betaglucan particles containing a payload of nanoembedded rifabutin for enhanced targeted delivery to macrophages. EXCLI journal, 2017. 16: p. 210-228, incorporated Rifabutin nanoprecipitates by incubation of the spray-dried GPs in a Rifabutin acidic aqueous solution followed by precipitation of the drug by addition of Tris buffer.

### Disclosure of the Invention

The formulations of the present invention, and the spray-drying method for preparation thereof, represent new GPs-based composites with controlled dissolution kinetics of insoluble and/or poorly-water soluble low-molecular-weight payloads. These new composites overcome the drawbacks of the background art and represent particles with uniform size and characteristic morphology appropriate for macrophage uptake, improved powder flowability, dispersibility in water, and dissolution kinetics which enable higher bioavailability of insoluble and/or poorly-water soluble low-molecular-weight compounds, such as medicaments (drugs) or food supplements. The technical effect of spray drying of glucan particles in presence of low-water soluble compound solution is the formation of amorphous low-water soluble compound inside the glucan particle. The result of the process is therefore glucan particle (microparticle) with incorporated amorphous compound, having significantly higher solubility and bioavailability than the same compound in crystalline form. Surprisingly, spray drying of glucan particles and poorly-water soluble compound solution results in formation of amorphous form of the compound inside (incorporated) of the glucan particles. Even though spray drying is known to be used for drying of temperature-sensitive materials, this method has never been used for amorphisation of the drug from an organic solvent inside GPs, water was rather used as a solvent, due to the hydrophilic character of beta glucans. Use of water, however, disables to dissolve poorly-water-soluble compounds and it does not preserve the size and characteristic morphology of glucan particles. Moreover, the microenvironment inside the GP during spray drying may exhibit specific conditions leading to very different physico-chemical behaviour of the substance being dried.

The present invention thus relates to novel composites of beta-glucan particles prepared from *Saccharomyces cerevisiae* (baker's yeast) and low-molecular-weight compounds, such as biologically active substances, which are insoluble or poorly soluble in water or in aqueous media. The insolubility or poor solubility in this application is related to the solubility in 10 mM phosphate-buffered saline (PBS) at 37 °C and pH 7.4 (water-based solution containing 9 g/L of NaCl in 10 mM disodium hydrogen phosphate). The poor aqueous solubility in the present application can thus be defined as solubility of at most 30 mg/mL in 10 mM PBS, measured at 37 °C and pH 7.4. The invention also provides for a method to produce these composites by spray drying and to a pharmaceutical composition and use thereof.

The low-molecular-weight compound in the present application is defined as a compound having its molecular mass of less than or equal to 5,000 Da, preferably a compound having its molecular mass of less than or equal to 1000 Da, more preferably in the range of from 100 to 600 Da.

The object of the present invention is therefore a method of production of a composite of yeast-derived beta-glucan particle with incorporated poorly-water-soluble low-molecular-weight compound, the poorly-water-soluble low-molecular-weight compound having solubility in 10 mM PBS of at most 30 mg/mL, measured at 37 °C and pH 7.4, and the weight ratio of the poorly-water-soluble low-molecular-weight compound to the yeast-derived beta-glucan particle is in the range of from 0.1·10⁻³ to 3, preferably from 0.1 to 2, more preferably from 0.2 to 1, most preferably from 0.25 to 0.5, wherein:
i) the poorly-water-soluble low-molecular-weight compound is dissolved in an organic solvent, selected from a group comprising ethanol, methanol, acetone, isopropanol, ethylacetate, dichloromethane, trichloromethane, chloroform, hexane, cyclohexane, heptane, toluene or mixtures thereof, preferably in a concentration up to 150 mg/ml, more preferably from 0.005 to 100 mg/ml, even more preferably from 5 to 50 mg/ml, most preferably from 10 to 30 mg/ml;
ii) beta glucan particles are added to the solution from step i) to form a suspension, preferably resulting in concentration of 50 mg to 4 g of beta-glucan particles per 100 ml of solution from step i), more preferably in concentration of from 200 mg to 3 g of beta-glucan particles per 100 ml of solution from step i), even more preferably in concentration of from 500 mg to 2 g of beta-glucan particles per 100 ml of solution from step i) most preferably in concentration of from 1 g to 1.5 g of beta-glucan particles per 100 ml of solution from step i);
iii) the suspension obtained in step ii) is spray dried under inert atmosphere to form the composite of yeast-derived beta-glucan particle with poorly-water-soluble low-molecular-weight compound incorporated in its amorphous form inside the glucan particles.

The resulting composite of yeast-derived beta-glucan particle with incorporated poorly-water-soluble low-molecular-weight compound can also contain the insoluble or poorly-water soluble low-molecular-weight compound partly within and partly outside of the glucan particles.

A surprising effect of the invention is the formulation of amorphous solid dispersions based on beta-glucan particles. The incorporation of insoluble or poorly-water soluble low-molecular-weight compounds into glucan particles promotes the amorphization of the compound, resulting in composites with faster dissolution rates, improved powder flowability and dispersibility in water, and accordingly, enhanced oral bioavailability. By fine-tuning of the spray-drying parameters and compositions of the composites, it is possible to produce preparations in which the drug is contained within the glucan particles, or composites in which the drug is partly within and partly outside of the glucan particles, and thus formulate preparations with different dissolution rates and/or biological responses, depending on the nature of the low-molecular-weight compound. Surprisingly, and contrary to the general knowledge in this field of art, it was possible to spray-dry hydrophilic materials (such as beta-glucans) from organic solvents, which are normally reserved for hydrophobic materials (such as poorly soluble drugs). The resulting spray-dried powder (GPs with incorporated poorly soluble low-molecular-weight compound) had much better properties, such as dissolution kinetics, particle size and morphology, powder rheology, and *in vitro* phagocytosis by macrophages. The size and morphology of the glucan particles is preserved, resulting in improved properties, such as powder flowability and water dispersibility.

In one embodiment, in step iii) the spray drying inlet temperature is from 30 to 350 °C, preferably the inlet temperature is from 50 to 150 °C.

In one embodiment (laboratory-scale spray dryer), the liquid feeding rate of the spray drying is between 1 and 20 mililiters per minute.

In one embodiment (laboratory-scale spray dryer), the inert gas flow rate ranges from 100 to 600 L/h. Nitrogen, argon or helium may be used as inert gases.

In another embodiment (pilot- and production-scale spray dryers), the liquid feeding rate is from 8 to 130 mililiters per minute and from 80 to 500 mililiters per minute, respectively, with proportionally higher inert gas flow rates.

In most preferred embodiment, the volumetric gas-to-liquid flow ratio (ratio of the gas feeding rate to liquid flow rate) is in the range from 50 to 10,000, more preferably from 100 to 5,000, even more preferably from 500 to 3,500, most preferably from 1000 to 3000, and the inlet temperature is from 30 to 250 °C, preferably the inlet temperature is from 50 to 150 °C. The enthalpy balance of the spray drying process is the following: In order to evaporate a given amount of liquid per unit of time, it is necessary to supply a certain amount of enthalpy per unit of time. The source of this enthalpy is the hot gas stream that enters the drying chamber along with the liquid stream. For a given inlet gas temperature, the outlet temperature is then the result of the ratio between these two streams: if the gas-to-liquid ratio is high, then there will be an excess of enthalpy and the temperature will remain high; on the other hand, if the gas-to-liquid ratio is low, then most of the enthalpy provided by the gas will be used for the evaporation of the liquid and the outlet temperature will drop. The gas-to-liquid ratio is independent of the size of the spray drying.

Preferably, the beta-glucan particles are prepared from *Saccharomyces cerevisiae.*

In more preferred embodiment, the beta glucan particles are prepared from *Saccharomyces cerevisiae* by the methodology described in Saloň, I., et al., *Suspension stability and diffusion properties of yeast glucan microparticles.* Food and Bioproducts Processing, 2016. **99:** p. 128-135. The preparation in based on a series of alkaline and acidic treatments, using aqueous hydroxide and aqueous inorganic acid at temperature above 50 °C, followed by washing steps with water and water miscible organic solvents. The final product is preferably freeze-dried.

In even more preferred embodiment, the beta-glucan particles are prepared by alkaline and acidic treatments of *Saccharomyces cerevisiae,* comprising the following steps:
a) natural or dried yeast is mixed with aqueous hydroxide, preferably in 1M NaOH or KOH, forming a suspension;
b) the suspension from step a) is homogenized and heated to at least 50 °C for at least 1 hour, preferably heated to 95 °C for 1 hour;
c) the suspension from step b) is centrifuged and the supernate is removed;
d) aqueous inorganic acid is added to the solid residue to adjust pH to about 4-5, and the suspension is heated to at least 50 °C for at least 2 hours;
e) the suspension from step d) is centrifuged and the supernate is removed;
f) the solid residue from step e) is washed with water and eventually water-miscible organic solvents, preferably selected from the group comprising isopropanol and acetone, and freeze-dried.

In one embodiment, first, baker's yeast is subjected to alkaline treatment three times. For that, 600 mL of 1 M NaOH solution are added to 150 grams of yeast. The suspension is heated to 90 °C and stirred with magnetic pill for one hour; then, it is centrifuged, and the supernatant is discarded. The pH of the slurry obtained after the alkaline treatments is adjusted between 4 and 5 by adding HCl solution (35%). The acidic suspension is stirred for 2 hours at 75 °C and centrifuged to discard the supernatant. Finally, the slurry is washed with deionized water (three times), isopropanol (four times) and acetone (two times), freeze-dried for two days and stored in a refrigerator for further use.

In one preferred embodiment, the poorly-water-soluble low-molecular-weight compound incorporated in the glucan particles according to the present invention is selected from a group comprising ibuprofen, curcumin, atorvastatin, diplacone, artemisinin, morusin, epigallocatechin gallate, resveratrol, acetylsalicylic acid, nilotinib, ellagic acid, acetyl-boswellic acid and amlodipine.

In one preferred embodiment, the low-water-soluble low-molecular-weight compound incorporated in the glucan particles is a drug for the treatment of pain, fever and inflammation, such as ibuprofen and acetylsalicylic acid; for the prevention of cardiovascular diseases, such as atorvastatin (lipid-lowering agent); for the treatment of chronic myelogenous leukemia (CML), such as nilotinib; for the treatment of high blood pressure and coronary artery disease, such as amlodipine; for the treatment of parasitic and infectious diseases, such as artemisinin and derivatives; and for the treatment of autoimmune diseases, such as artemisinin and derivatives. In one preferred embodiment, the low-water-soluble low-molecular-weight compound incorporated in the glucan particles is an antioxidant and/or agent with anti-inflammatory activity (such as curcumin, diplacone, morusin, epigallocatechin gallate, resveratrol, ellagic acid, and acetyl-boswellic acid), which can be used as a food supplement. Moreover, curcumin, besides antioxidant and anti-inflammatory, exhibits immunomodulatory, antibacterial, antiviral, antifungal, and anti-mutagenic activity, and can be used as food and cosmetic colorant.

Another object of the present invention is a composite of yeast-derived beta glucan particle with incorporated poorly-water-soluble low-molecular-weight compound, the poorly-water-soluble low-molecular-weight compound having solubility in 10 mM PBS of at most 30 mg/mL, measured at 37 °C and pH 7.4, and the weight ratio of the poorly-water-soluble low-molecular-weight compound to the yeast-derived beta-glucan particle is in the range of from 0.1·10⁻³ to 3, preferably from 0.1 to 2, more preferably from 0.2 to 1, most preferably from 0.25 to 0.5, obtainable by the above described method according to the present invention, wherein the poorly-water-soluble low-molecular-weight compound is incorporated inside the yeast-derived beta-glucan particle in an amorphous form.

In one preferred embodiment of the composite according to the present invention, the low-water-soluble low-molecular-weight compound incorporated inside the yeast-derived beta glucan particle in amorphous form is selected from the group comprising ibuprofen, curcumin, atorvastatin, diplacone, artemisinin, morusin, epigallocatechin gallate, resveratrol, acetylsalicylic acid, nilotinib, ellagic acid, acetyl-boswellic acid and amlodipine.

In one preferred embodiment, the low-water-soluble drug incorporated in the glucan particles is a drug for the treatment of pain, fever and inflammation, such as ibuprofen and acetylsalicylic acid; for the prevention of cardiovascular diseases, such as atorvastatin (lipid-lowering agent); for the treatment of chronic myelogenous leukemia (CML), such as nilotinib; for the treatment of high blood pressure and coronary artery disease, such as amlodipine; for the treatment of parasitic and infectious diseases, such as artemisinin and derivatives; and for the treatment of autoimmune diseases, such as artemisinin and derivatives.

In one preferred embodiment, the low-water-soluble low-molecular-weight compound incorporated in the glucan particles is an antioxidant and/or agent with anti-inflammatory activity (such as curcumin, diplacone, morusin, epigallocatechin gallate, resveratrol, ellagic acid, and acetyl-boswellic acid), which can be used as a food supplement. Moreover, curcumin, besides antioxidant and anti-inflammatory, exhibits immunomodulatory, antibacterial, antiviral, antifungal, and anti-mutagenic activity, and can be used as food and cosmetic colorant.

Another object of the present invention is a pharmaceutical composition, which comprises the composite according to the present invention, wherein the poorly-water soluble low-molecular-weight compound incorporated in the GPs is a medicament, and wherein the pharmaceutical composition further comprises at least one pharmaceutically acceptable carrier, selected from a group comprising fillers, such as sugars, for example lactose, sucrose, mannitol or sorbitol, cellulose preparations and/or calcium phosphates, for example tricalcium diphosphate, or calcium hydrogen phosphate, and furthermore binders, such as starches, for example maize, wheat, rice or potato starch, methylcellulose, hydroxypropylmethylcellulose, sodium carboxymethylcellulose and/or polyvinylpyrrolidine, and/or, if desired, disintegrants, such as the above mentioned starches, and furthermore carboxymethyl-starch, cross-linked polyvinylpyrrolidone, alginic acid or a salt thereof, such as sodium alginate; stabilizers; excipients, in particular flow regulators and lubricants, for example salicylic acid, talc, stearic acid or salts thereof, such as magnesium stearate or calcium stearate, and/or polyethylene glycol, or derivatives thereof.

In one embodiment, the pharmaceutical composition further comprises a poorly-water-soluble low-molecular-weight medicament in crystalline form, not encapsulated in glucan particles. The poorly-water-soluble low-molecular-weight medicament in crystalline form has solubility in 10 mM PBS of at most 30 mg/mL, measured at 37 °C and pH 7.4. Preferably, it is selected from a group comprising ibuprofen, curcumin, atorvastatin, diplacone, artemisinin, morusin, epigallocatechin gallate, resveratrol, acetylsalicylic acid, nilotinib, ellagic acid, acetyl-boswellic acid and amlodipine.

The presence of amorphous active compound (medicament) in the glucan particle and of the crystalline active compound outside the glucan particle in the pharmaceutical composition enables to adjust and control dissolution kinetics and bioavailability of the poorly-water-soluble active compound used.

In one embodiment, the medicament in crystalline form is the same as the medicament in amorphous state incorporated inside the glucan particle composite present in the pharmaceutical composition.

Another object of the present invention is the use of the composite and/or the pharmaceutical composition according to the present invention as a carrier of the poorly-water-soluble low-molecular-weight drug in medicine.

Another object of the present invention is the use of the pharmaceutical composition according to the present invention as a medicament with controlled release.

Another object of the present invention is the use of the composite according to the present invention, wherein the poorly-water soluble low-molecular compound is a food supplement, as a carrier of the food supplement. More preferably, the low-water-soluble low-molecular-weight compound incorporated in the glucan particles is a food supplement with antioxidant and/or anti-inflammatory activity such as curcumin, diplacone, morusin, epigallocatechin gallate, resveratrol, ellagic acid, and acetyl-boswellic acid; with immunomodulatory, antibacterial, antiviral, antifungal, and anti-mutagenic activity, such as curcumin.

### Brief Description of Figures

Fig. 1: The morphology of the microparticles produced in Example 2, evaluated by Scanning Electron Microscopy (SEM) using a Jeol JCM-5700 microscope.
Fig. 2: Relative drug content of composites of Example 2.
Fig. 3: X-ray diffraction, evaluating the crystallinity of samples from Example 2, using a PANaytical X'Pert PRO with High Score Plus diffractometer.
Fig. 4: The morphology of the microparticles produced in Example 3, evaluated by Scanning Electron Microscopy (SEM) using a Jeol JCM-5700 microscope. Arrows are showing curcumin found outside of the glucan particles.
Fig. 5: Fluorescent microscopy of composites of Example 3.
Fig. 6: Confocal microscopy of composites of Example 3.
Fig. 7: Relative drug content of composites of Example 3.
Fig. 8: X-ray diffraction, evaluating the crystallinity of the samples produced in Example 3, using a PANaytical X'Pert PRO with High Score Plus diffractometer.
Fig. 9: The morphology of the microparticles produced in Example 4, evaluated by Scanning Electron Microscopy (SEM) using a Jeol JCM-5700 microscope. Circles and arrows are showing crystals found in the samples.
Fig. 10: X-ray diffraction patterns of ibuprofen and spray-dried glucan particles (SD-GP, IBU-GP-0.1, IBU-GP-0.2, IBU-GP-0.5, IBU-GP-1.0, IBU-GP-2.0), evaluating the crystallinity of the samples produced in Example 4, using a PANaytical X'Pert PRO with High Score Plus diffractometer.
Fig. 11: Dissolution kinetics of crude micronized ibuprofen, (IBU+ASA)/GP composites, and crude acetylsalicylic acid, produced according to Example 5.
Fig. 12: Dissolution kinetics of crude amlodipine, and AML/GP composites, produced according to Example 6.
Fig. 13: Comparison of wettability and dispersion of IBU/GP composites (left vial), produced according to Example 7, and crude ibuprofen (right vial), immediately after contact with water (a) and mildly shaken after 5 minutes (b).
Fig. 14: Dissolution kinetics of crude micronized ibuprofen, IBU/GP composites, and mixtures of them, produced according to Example 7.
Fig. 15: Powder rheology results for crude atorvastatin, ATO/GP composites produced by spray drying and by rotary evaporation, according to Example 8: (a) Crude atorvastatin; (b) ATO/GP-RE; (c) ATO/GP-SD.
Fig. 16: The morphology of the composites produced in Comparative Example 9, evaluated by Scanning Electron Microscopy (SEM) using a Jeol JCM-5700 microscope: ATO/GP composites, magnification 500x (a) and 2000x (b); ATO/PVP composites, magnification 500x (c); ATO/SLP composites, magnification 500x (d).
Fig. 17: The morphology of the composites produced in Comparative Example 10, evaluated by Scanning Electron Microscopy (SEM) using a Jeol JCM-5700 microscope: GP-EtOH, magnification 500x (a) and 2000x (b); GP-EtOH/water, magnification 500x (c) and 2000x (d); and GP-water, magnification 500x (e) and 2000x (f).
Fig. 18: Particle size distributions of the composites produced in Comparative Example 10, evaluated by static light scattering using Horiba Partica LA 950/S2 equipment.
Fig. 19: Phagocytosis of macrophages according to Example 10, observed after 3 hours using an Olympus Fluoview FV1000 confocal system for: GP/CC-EtOH sample observed using objective 40x with zoom mode (a), and with excitation wavelength 405 nm and zoom mode (b); GP/NR-EtOH sample observed using objective 60x (c), and with excitation wavelength 550 nm (d).

### Examples

The invention is further illustrated by, but not limited to, specific examples.

### Example 1 - General method of preparation of composites of yeast-derived betaglucan particles and poorly-water-soluble low-molecular-weight compound

The composites of yeast-derived beta glucan particles and poorly-water-soluble low-molecular-weight compounds according to the present invention were produced by spray drying using a Mini Spray Dryer B-290 from Büchi operated in inert loop under N₂ atmosphere, and equipped with a 2-fluid nozzle (0.7 mm of diameter) or an ultrasonic package (ultrasonic nozzle and controller). A solution of the poorly-water-soluble low-molecular-weight compound in an organic solvent (such as ethanol, methanol, acetone, isopropanol, dichloromethane or mixtures thereof) with desired concentration (typically in the range of from 0.5 to 20 mg/mL) is prepared, and glucan particles are added to the low-molecular-weight compound solution to form a suspension, containing from 2 to 40 mg of glucan particles per 1 ml of the suspension. The resulting suspension is spray dried under inert atmosphere, typically nitrogen, and using previously defined parameters. The spray drying process promotes the rapid evaporation of the organic solvent and the subsequent precipitation of the drug within or within and outside the glucan particles. The spray-drying parameters can be changed to produce different composite formulations. The inlet temperature is selected based on the boiling point of the organic solvent and/or thermal degradation properties of the starting materials. Feeding rate and gas flow rate mainly influence droplet size. Feeding rate in the experiments varied between 1 and 20 mililiters per minute, and the gas flow rate from 100 to 600 L/h.

The beta glucan particles for the experiment were prepared from *Saccharomyces cerevisiae* based on the methodology described in Saloň, I., et al., *Suspension stability and diffusion properties of yeast glucan microparticles.* Food and Bioproducts Processing, 2016. **99:** p. 128-135. First, baker's yeast was subjected to alkaline treatment. For that, 600 mL of 1 M NaOH solution were added to 150 grams of yeast. The suspension was heated to 90 °C and stirred with magnetic pill for one hour; then, it was centrifuged, and the supernatant was discarded. The alkaline treatment was repeated three times. The pH of the slurry obtained after the alkaline treatments was adjusted between 4 and 5 by adding HCl solution (35%). The acidic suspension was stirred for 2 hours at 75 °C and centrifuged to discard the supernatant. Finally, the slurry was washed with deionized water (three times), isopropanol (four times) and acetone (two times), freeze-dried for two days and stored in a refrigerator for further use.

Formulation of yeast-derived beta-glucan particles and insoluble or poorly-water soluble low-molecular-weight compounds:
Various formulations of yeast-derived beta-glucan particles and insoluble or poorly-water soluble drugs were prepared by using different low-molecular-weight compounds and/or combination of them, different solvents and/or combination of them, and varying the drug/GP mass ratios. The solvent may be, for example, ethanol, methanol, acetone, isopropanol, dichloromethane or other organic solvents, and/or mixtures of them. The scale of the experiment may vary from milligrams to hundreds of grams of the glucan particles, thus covering the industrial production. The weight of the poorly-water soluble low-molecular-weight compound is then given by the desired fraction of the drug in the composite, which can range from 0.1 to over 3.0. The ratio between the weight of the glucan particles and the volume of the solvent may range from tens of milligrams to tens of grams of particles per liter of solvent according to the desired properties of the composites. Examples of the preparations used for testing are given in the following Table 1.

**Table 1:**

| **Model low-molecular-weight compound** | **Solvent used** | **Low-molecular-weight compound concentratio n (mg/mL)** | **GPs concentratio n (mg/mL)** | **Low-molecular-weight compound/G Ps weight ratio** | **Spray drying conditions *** |
|---|---|---|---|---|---|
| Ibuprofen | Ethanol | 1.00 | 10.0 | 0.10 | 2FN, S and L |
| Ibuprofen | Ethanol | 2.00 | 20.0 | 0.10 | 2FN, S and L |
| Ibuprofen | Ethanol | 2.00 | 10.0 | 0.20 | 2FN, S and L |
| Ibuprofen | Ethanol | 5.00 | 10.0 | 0.50 | 2FN, S and L |
| Ibuprofen | Ethanol | 10.0 | 10.0 | 1.0 | 2FN, S and L |
| Ibuprofen | Ethanol | 20.0 | 10.0 | 2.0 | 2FN, S and L |
| Ibuprofen | Ethanol | 5.00 | 20.0 | 0.25 | USN |
| Curcumin | Ethanol | 0.0100 | 20.0 | 0.50 × 10⁻³ | USN |
| Curcumin | Ethanol | 0.0200 | 20.0 | 1.0 × 10⁻³ | USN |
| Curcumin | Ethanol | 0.100 | 20.0 | 5.0 × 10⁻³ | USN |
| Curcumin | Ethanol | 0.200 | 20.0 | 0.010 | USN |
| Curcumin | Ethanol | 1.00 | 20.0 | 0.050 | 2FN (S and L), USN |
| Curcumin | Ethanol | 2.00 | 20.0 | 0.10 | USN |
| Curcumin | Ethanol | 3.60 | 20.0 | 0.18 | 2FN, L |
| Curcumin | Ethanol | 4.00 | 20.0 | 0.20 | USN |
| Curcumin | Ethanol | 6.00 | 20.0 | 0.30 | USN |
| Diplacone | Ethanol | 2.50 × 10⁻³ | 20.0 | 0.13 × 10⁻³ | USN |
| Diplacone | Ethanol | 0.0130 | 20.0 | 0.63 × 10⁻³ | USN |
| Diplacone | Ethanol | 0.127 | 20.0 | 6.3 × 10⁻³ | USN |
| Artemisinin | Ethanol | 8.40 × 10⁻³ | 20.0 | 0.42 × 10⁻³ | USN |
| Epigallocatech in gallate | Ethanol | 0.0140 | 20.0 | 0.68 × 10⁻³ | USN |
| Resveratrol | Ethanol | 6.80 × 10⁻³ | 20.0 | 0.34 × 10⁻³ | USN |
| Ellagic acid | Ethanol | 9.00 × 10⁻³ | 20.0 | 0.45 × 10⁻³ | USN |
| Morusin | Ethanol | 0.0130 | 20.0 | 0.63 × 10⁻³ | USN |
| Acetyl-boswellic acid | Ethanol | 0.50 | 10.0 | 0.050 | USN |
| Atorvastatin | Ethanol | 0.0170 | 20.0 | 0.85 × 10⁻³ | USN |
| Atorvastatin | Ethanol | 0.0330 | 20.0 | 1.7 × 10⁻³ | USN |
| Atorvastatin | Ethanol | 0.167 | 20.0 | 8.4 × 10⁻³ | USN |
| Atorvastatin | Ethanol | 1.00 | 20.0 | 0.050 | USN |
| Atorvastatin | Ethanol | 2.00 | 20.0 | 0.10 | USN |
| Atorvastatin | Ethanol | 3.00 | 20.0 | 0.15 | USN |
| Atorvastatin | Methanol | 1.00 | 20.0 | 0.05 | USN |
| Amlodipine | Ethanol | 5.00 | 20.0 | 0.25 | USN |
| Amlodipine | DCM | 5.00 | 20.0 | 0.25 | USN |
| Amlodipine | DCM/Ethanol (1/1 V/V) | 5.00 | 20.0 | 0.25 | USN |
| Ibuprofen/ Acetylsalicylic acid (1/1) | Ethanol | 5.00 | 20.0 | 0.25 | USN |
| Nilotinib | Methanol/DC M (7/3 V/V) | 3.0 | 20.0 | 0.15 | 2FN |
| Nilotinib | Methanol/DC M (7/3 V/V) | 2.0 | 20.0 | 0.10 | 2FN |
| Nilotinib | Methanol/DC M (7/3 V/V) | 1.0 | 20.0 | 0.050 | 2FN |
| Nilotinib | Methanol/DC M (7/3 V/V) | 0.20 | 20.0 | 0.010 | 2FN |
| Nilotinib | Methanol/DC M (7/3 V/V) | 0.0020 | 20.0 | 0.10 × 10⁻³ | 2FN |
| Nilotinib | Methanol/DC M (7/3 V/V) | 0.00040 | 20.0 | 0.020 × 10⁻³ | 2FN |

| | | | | | |
|---|---|---|---|---|---|
| *2FN: 2-fluid nozzle; S: *small*-droplet configuration; L: *large*-droplet configuration; USN: ultrasonic nozzle (with which is possible to produce *extra-large* droplets). | | | | | |

### Example 2 - Preparation of composites with different processing conditions

Composites of yeast-derived beta glucan particles with incorporated poorly-water-soluble low-molecular-weight compound were prepared according to the procedure of Example 1, using ibuprofen (IBU) as the poorly-water-soluble low-molecular-weight compound model, with a fixed IBU-to-GP weight ratio of 0.1. Different samples were produced by changing the processing conditions, namely initial solid content and spray-drying parameters (feeding rate and flow rate). The initial solid contents tested were 10 and 20 mg/mL, i.e. 1 or 2 grams of glucan particles were added in 100 mililiters of ibuprofen solution with concentration of 1 mg/mL or 2 mg/mL respectively. Ethanol was used as the organic solvent.

The prepared 100-mL suspensions were spray-dried using the 2-fluid nozzle. In order to evaluate the influence of droplet size in the final composites, two different set of operating conditions were tested. The first one (small droplets) consisted of 3.5 mL/min feeding rate and 600 L/h (50%) N₂ flow rate; the second set (large droplets) consisted in 7.0 mL/min feeding rate and 473 L/h (40%) N₂ flow rate. In both cases, the outlet temperature was kept constant at (75 ± 2) °C, for which the inlet temperature was varied between 120 to 130 °C.

The samples are labelled as:
- *S10:* Composites prepared with initial solid content 10 mg/mL and *small* droplets set of parameters.
- *S20:* Composites prepared with initial solid content 20 mg/mL and *small* droplets set of parameters.
- *L10*: Composites prepared with initial solid content 10 mg/mL and *large* droplets set of parameters.
- *L20*: Composites prepared with initial solid content 20 mg/mL and *large* droplets set of parameters.

### Morphology of the composites

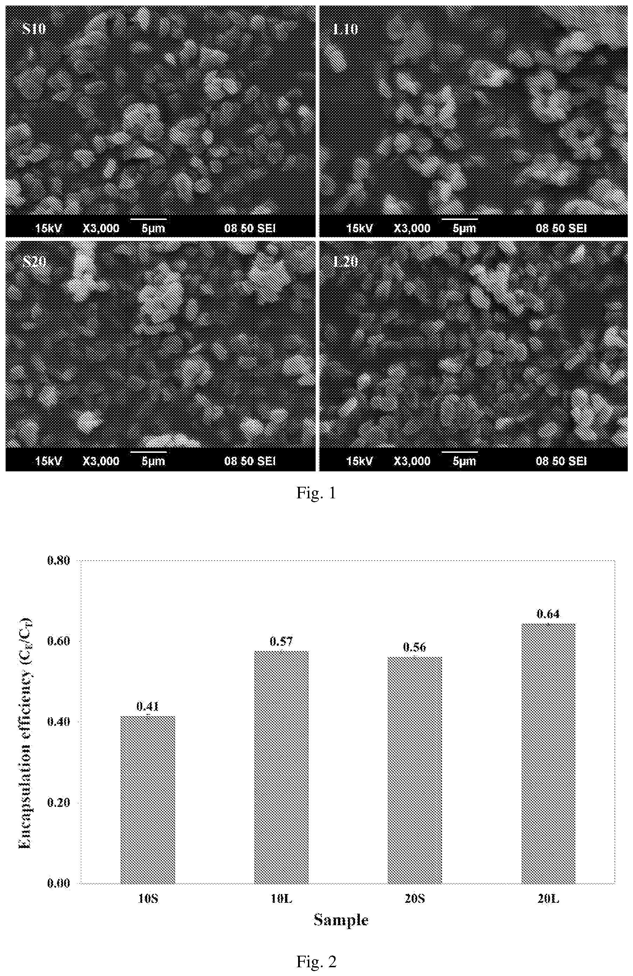

The morphology of the produced microparticles (Fig. 1) was evaluated by Scanning Electron Microscopy (SEM) using a Jeol JCM-5700 microscope. Before the SEM analysis, the samples were coated with a 5-nm gold layer using an Emitech K550X sputter coating equipment.

The glucan particles present the typical ellipsoidal morphology with 2-4 µm particle size, exhibiting a wrinkled surface that can be attributed to the hydrolysis of the yeast outer cell wall and intercellular components, product of the alkaline and acid treatments. No evidence of ibuprofen outside of the glucan particles is observed.

### Encapsulation efficiency

For the determination of the encapsulation efficiency (Fig. 2), ibuprofen was extracted from the produced IBU/GP composites by adding 10.0 mg of the microparticles to a 10.0 mL of phosphate buffer solution (pH 7.4). The dispersions were placed in an ultrasonication bath for 10 min to guarantee the complete extraction of the ibuprofen from the glucan particles. Afterwards, the glucan particles were separated by centrifugation (5 min at 7000 rpm), and 500 µL of supernatant were collected. The concentration was evaluated by high-performance liquid chromatography (HPLC) with UV detection (Agilent), coupled with C18 column (100 mm × 4.6 mm, 5 µm) and mobile phase consisting of 0.01 M ammonium phosphate buffer (pH 2.0) and acetonitrile (60%). The encapsulation efficiency of the IBU/GP composite microparticles was calculated as the experimental concentration of active compound (C_{E}), measured by HPLC, divided by the theoretical concentration (C_{T}) of ibuprofen in the composites.

Significantly higher encapsulation efficiencies (C_{E}/C_{T}) were obtained for the samples produced with large-droplets settings (10L and 20L) when compared with the samples produced with small-droplet settings. In addition, higher encapsulation efficiencies were obtained for the samples produced from dispersions with higher solid content.

### X-Ray Diffraction

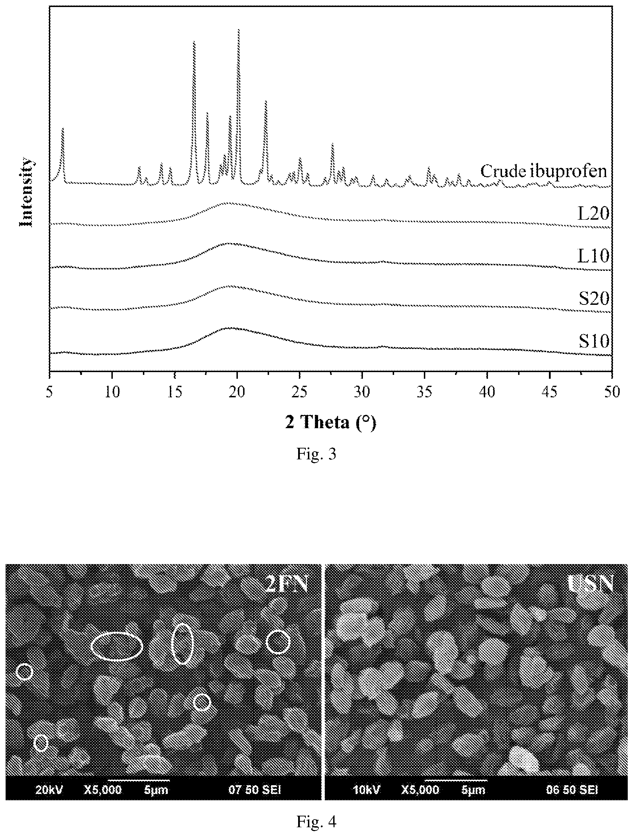

Crystallinity of the samples (Fig. 3) was evaluated by recording the diffraction intensities of the produced microparticles from 5° to 50° 2θ angle using a PANaytical X'Pert PRO with High Score Plus diffractometer. Unlike the micronized crude ibuprofen, all IBU/GP composites produced are completely amorphous.

### Example 3 - Preparation of composites with different spray-drying nozzles

Composites of yeast-derived beta-glucan particles with incorporated poorly-water-soluble low-molecular-weight compound were prepared according to the procedure of Example 1 using curcumin (CC) as a poorly-water-soluble low-molecular-weight compound model, with a fixed CC-to-GP weight ratio of 0.05. For that, 50-mL suspensions (20 mg/mL) were prepared by adding 1 gram of glucan particles in 50 mililiters of curcumin solution with concentration of 1 mg/mL of ethanol. Afterwards, the suspensions were spray-dried using different spray-drying nozzles, namely a 2-fluid nozzle (0.7 mm of diameter) and the ultrasonic nozzle. The different nozzles can mainly influence droplet size and morphology of the samples.

For the sample spray dried using the 2-fluid nozzle (labelled *2FN*)*,* the operating conditions used consisted of 3.5 mL/min feeding rate and 473 L/h (40%) N₂ flow rate. For the sample spray-dried using the ultrasonic nozzle (labelled *USN*)*,* the operating conditions consisted of 3.5 mL/min feeding rate, 246 L/h (20%) N₂ flow rate, and 1.8 watts (ultrasonic nozzle power). In both cases the inlet temperature was 120 °C, for which the outlet temperature was (75 ± 2) °C.

### Morphology of the composites

The morphology of the produced microparticles (Fig. 1) was evaluated by Scanning Electron Microscopy (SEM) using a Jeol JCM-5700 microscope. Before the SEM analysis, the samples were coated with a 5-nm gold layer using an Emitech K550X sputter coating equipment. Besides the typical ellipsoidal, wrinkled morphology of the glucan particles, another type of particles with spherical morphology were observed and attributed to curcumin precipitated outside of the glucan particles. The curcumin outside of the glucan particles is much more evident in the 2FN sample than in the USN sample.

### Fluorescent and confocal microscopy

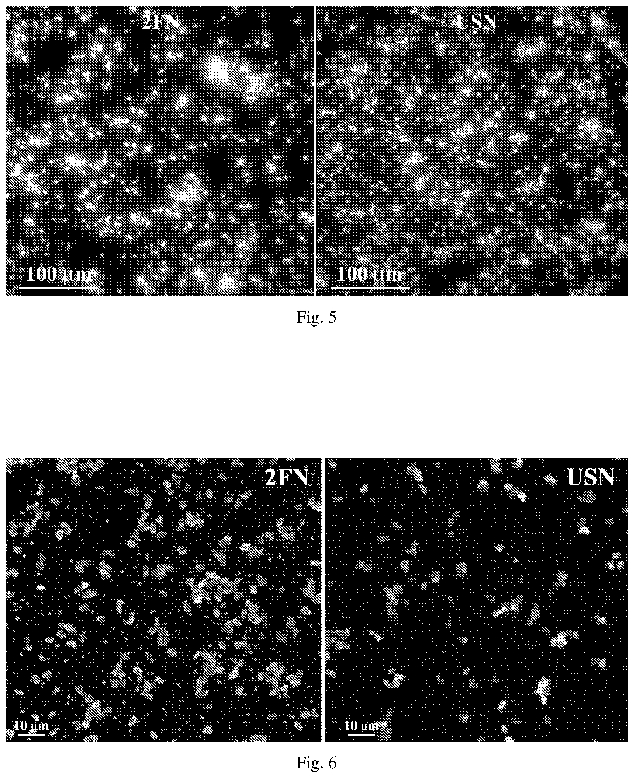

Samples were analyzed by fluorescent (Fig. 5) and confocal microscopy (Fig. 6) using an Olympus Fluoview FV1000 confocal system (488 nm excitation wavelength). From fluorescent microscopy images is possible to observe that the loading of curcumin in the glucan particles was uniform for both samples (2FN and USN). In the confocal microscopy images, a large amount of curcumin particles outside of the glucan particles (small dots) are observed in the 2FN-sample, whereas in the USN-samples such particles are not observed, evidencing that all the curcumin was encapsulated inside the glucan particles.

### Encapsulation efficiency

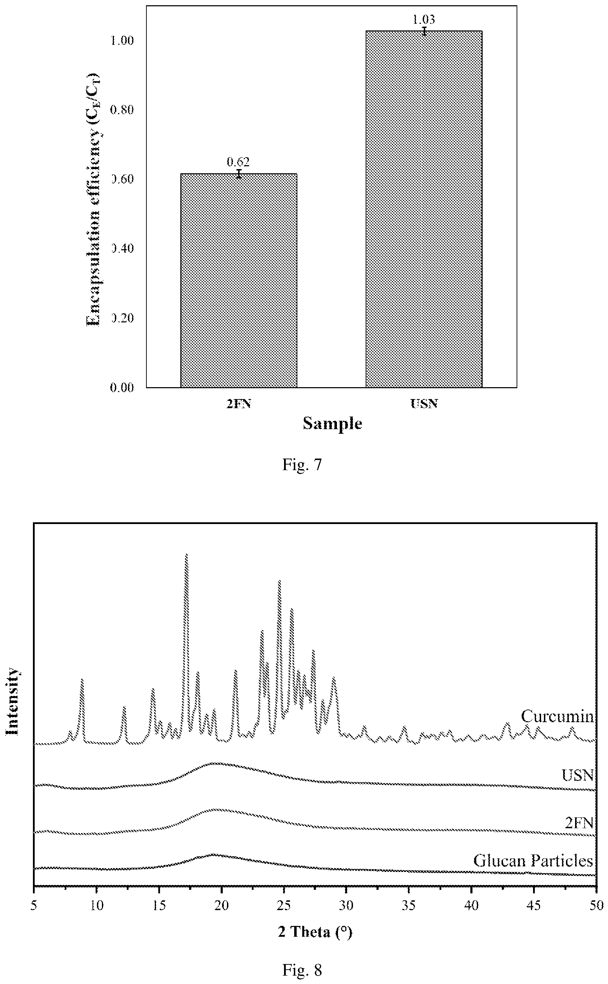

The curcumin content of the CC/GP composite microparticles (Fig. 7) was calculated as the experimental concentration (C_{E}) of curcumin, measured by UV-Vis spectrophotometry, divided by the theoretical concentration (C_{T}) of curcumin. For the determination of the experimental concentration, curcumin was extracted from the produced CC/GP composites by adding 5.0 mg of the microparticles to 10.0 mL of methanol. The dispersions were placed in an ultrasonication bath for 10 min to guarantee the complete extraction of the curcumin from the glucan particles. Afterwards, the glucan particles were separated by centrifugation (10 min at 5000 rpm), and 3.0 mL of supernatant were filtered and placed into a spectrophotometer cuvette. Dilutions were done when necessary. Absorbance (λ = 425 nm) was measured by UV-Vis spectrophotometry, using a Specord 205 BU UV-Vis spectrophotometer, and related to the concentration of curcumin using a calibration curve previously plotted.

The encapsulation efficiency was approximately 100% for the USN-sample and 61.5% for the 2FN-sample. The 40% difference is attributed to losses of curcumin that precipitated outside of the glucan particles in the case of the 2FN-sample. Such curcumin particles are very small; therefore, there is a high possibility that they were not collected in the cyclone of the spray dryer, causing the losses along the spray dryer.

### X-Ray Diffraction

Crystallinity of the samples (Fig. 8) was evaluated by recording the diffraction intensities of the produced microparticles from 5° to 50° 2θ angle using a PANaytical X'Pert PRO with High Score Plus diffractometer. Unlike the pure curcumin, all CC/GP composites produced are completely amorphous.

### Example 4 - Preparation of the composites with increasing low-molecular-weight compound loading

Composites of glucan particles and ibuprofen (IBU), as poorly-water soluble model low-molecular-weight compound, were prepared according to the procedure of Example 1, considering increasing IBU/GP mass ratios (0.1, 0.2, 0.5, 1.0 and 2.0). For that, 100-mL ibuprofen solutions were prepared with concentrations 0.1, 0.2, 0.5, 1.0 and 2.0 % (w/v), using ethanol as organic solvent. Afterwards, 1.0 g of glucan particles was added to each solution and dispersed using an IKA^{®} T10 basic ultra-turrax for 5 minutes before spray-drying. The dispersions were incubated overnight at room temperature before spray-drying. The samples are labelled as IBU-GP-0.1, IBU-GP-0.2, IBU-GP-0.5, IBU-GP-1.0 and IBU-GP-2.0 respectively. An analogous unloaded sample referred as "SD-GP" was also prepared and spray-dried.

The 100-mL samples were spray-dried using the Mini Spray Dryer B-290 equipped with the 2-fluid nozzle (0.7 mm of diameter) and operated in inert loop under N₂ atmosphere. Two different set of operating conditions were used. The first one (small droplets) consisted of 120 °C inlet temperature, 3.5 mL/min feed rate and 600 L/h (50%) N₂ flow rate. The second set of operating conditions (large droplets) was: 130 °C inlet temperature, 7.0 mL/min feed rate and 473 L/h (40%) N₂ flow rate. In both cases, the outlet temperature was from 66 to 72 °C.

### Morphological characterization

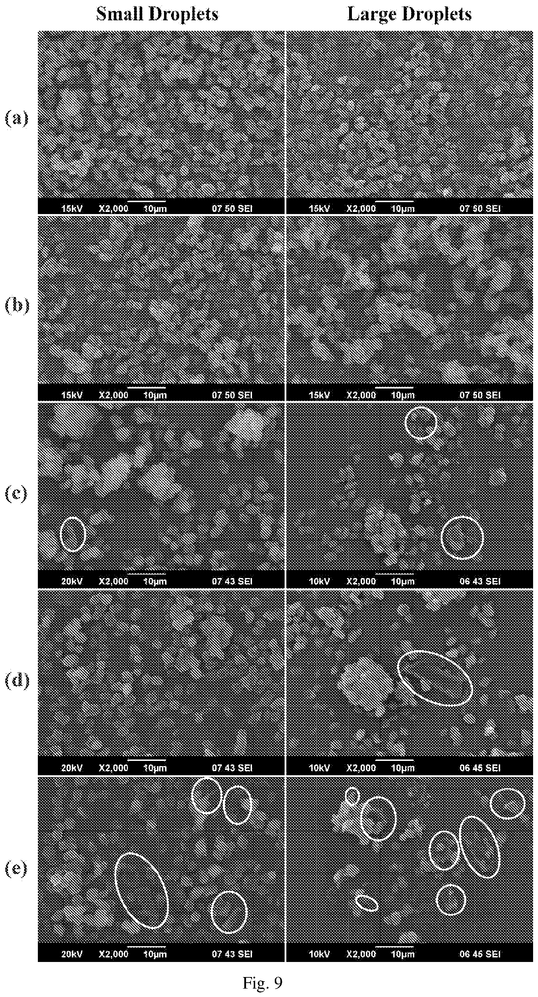

The morphology of the produced microparticles (Fig. 9) was evaluated by Scanning Electron Microscopy (SEM) using a Jeol JCM-5700 microscope. Before the SEM analysis, the samples were coated with a 5-nm gold layer using an Emitech K550X sputter coating equipment. The presence of ibuprofen crystals outside of the glucan particles was observed in the samples with higher IBU content (IBU/GP mass ratio ≥ 0.5). The crystals appeared larger in size and quantity in the samples produced with the large-droplet spray-drying settings.

### X-Ray Diffraction

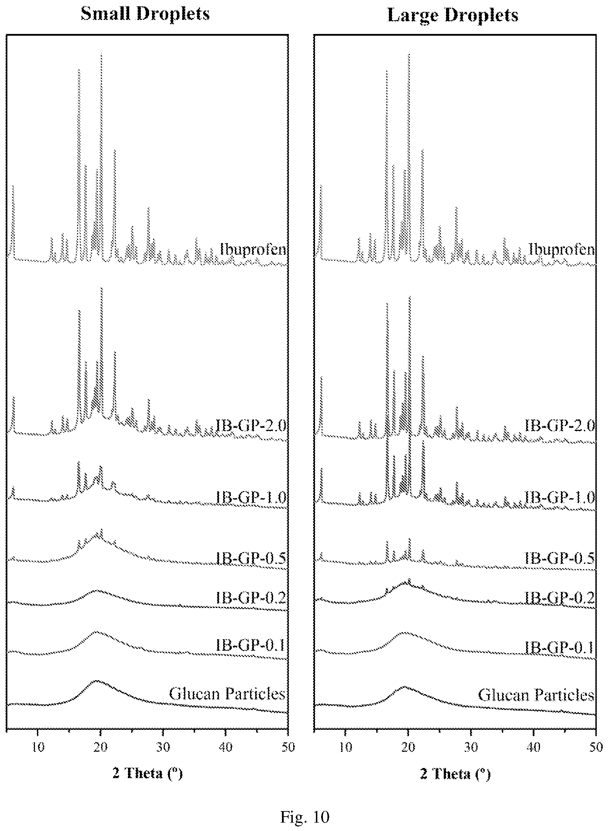

Crystallinity of the samples (Fig. 10) was evaluated by recording the diffraction intensities of the produced microparticles from 5° to 50° 2θ angle using a PANaytical X'Pert PRO with High Score Plus diffractometer. A tendency of crystallinity to increase with IBU content and droplet size was observed, in accordance to the SEM observations.

### Example 5 - Preparation of composites with combination of more than one poorly-water soluble low-molecular-weight compounds

Composites of glucan particles and two different poorly-water soluble model low-molecular-weight compounds, ibuprofen (IBU) and acetylsalicylic acid (ASA), were prepared according to the procedure of Example 1. The composites were prepared considering a drug/GP mass ratio of 25% (IBU/GP = 12.5% wt. and ASA/GP = 12.5% wt.) and using ethanol as organic solvent. For that, 50-mL of drug solution were prepared by dissolving 125 mg of IBU and 125 mg of ASA, using ethanol as common organic solvent. Afterwards, 1.0 g of glucan particles was added to the drug solution and dispersed using an IKA^{®} T10 basic ultra-turrax for 5 minutes before spray drying. The sample was spray-dried using the Mini Spray Dryer B-290 equipped with the ultrasonic nozzle and operated in inert loop under N₂ atmosphere. The operating conditions used consisted of 120°C inlet temperature, 5.0 mL/min feed rate, 246 L/h (20%) N₂ flow rate and 2.0 W power outlet at nozzle. The outlet temperature was 76 °C.

### Dissolution kinetics

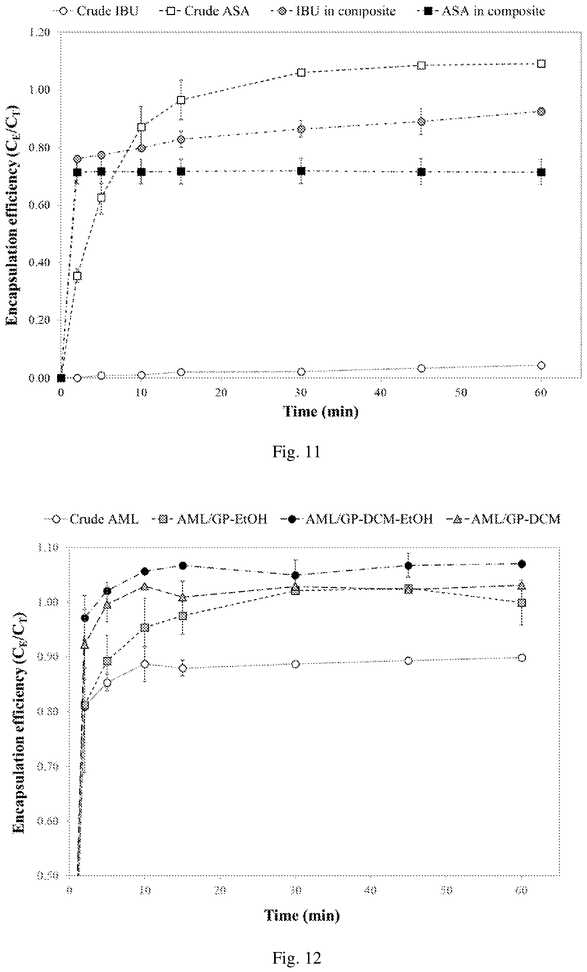

Dissolution tests (Fig. 11) were performed for crude micronized ibuprofen, crude acetylsalicylic acid and for the produced (IBU+ASA)/GP composite particles, in powder form and using 10 mM HCl (pH 2.0) as dissolution medium. For that, 20.0 mg of crude drug (IBU or ASA) or 100.0 mg of (IBU+ASA)/GP composite, were added to 200 mL of continuously stirred dissolution medium (for a maximum concentration of 0.1 mg of drug per ml of medium). The mixtures were continuously stirred at 250 rpm and room temperature in a 250-mL beaker. At pre-defined time-points (ranging from 0 to 60 min), 500 µL of sample were collected, centrifuged and filtered (200-nm pore size filtration membrane), and the concentration was evaluated by high-performance liquid chromatography (HPLC) with UV detection (Agilent), coupled with C18 column (100 mm × 4.6 mm, 5 µm) and mobile phase consisting of 0.01 M ammonium phosphate buffer (pH 2.0) and acetonitrile, in gradient according to the Table 2:

**Table 2:**

| Time (min) | % A | % B | Flow (ml/min) |
|---|---|---|---|
| 0.0 | 80 | 20 | 1 |
| 3.0 | 80 | 20 | 1 |
| 3.5 | 40 | 60 | 1 |
| 6.5 | 40 | 60 | 1 |
| 7.0 | 80 | 20 | 1 |
| 9.0 | 80 | 20 | 1 |

After 2 minutes, both IBU and ASA encapsulated in the glucan particles were completely dissolved, whereas crude IBU and crude ASA exhibit slower dissolution rates.

### Example 6 - Preparation of composites with different pure organic solvents and combinations of them

Composites of glucan particles and amlodipine (AML), as poorly-water soluble model low-molecular-weight compound, were prepared according to the procedure of Example 1, considering an AML/GP mass ratio of 25% and using different organic solvents and combinations of them. For that, 50-mL amlodipine solutions were prepared with concentration 5 mg/mL, using ethanol, dicloromethane (DCM) and a mixture of ethanol/DCM (50/50) as organic solvents. Afterwards, 1.0 g of glucan particles was added to each solution and dispersed using an IKA^{®} T10 basic ultra-turrax for 5 minutes before spray drying.

The samples are labelled:
- AML/GP-EtOH: For the composites prepared using 100% ethanol as solvent.
- AML/GP-DCM-EtOH: For the composites prepared using 50% DCM and 50% ethanol as solvents.
- AML/GP-DCM: For the composites prepared using 100% DCM as solvent.

The three samples were spray-dried using the Mini Spray Dryer B-290 equipped with the ultrasonic nozzle and operated in inert loop under N₂ atmosphere. The operating conditions used consisted of 120°C, 90 °C and 80 °C inlet temperature respectively for AML/GP-EtOH, AML/GP-DCM-EtOH and AML/GP-DCM samples. In all cases, 5.0 mL/min feeding rate, 246 L/h (20%) N₂ flow rate and 2.4 W power outlet at nozzle were set. The outlet temperature was 76 °C, 56 °C and 54 °C, respectively.

### Dissolution kinetics

Dissolution tests (Fig. 12) were performed for crude amlodipine and for the produced AML/GP composite particles, in powder form and using distilled water as dissolution medium. For that, 20.0 mg of crude amlodipine or 100.0 mg of composites were added to 200 mL of dissolution medium (for a maximum concentration of 0.1 mg of amlodipine per ml of medium). The mixtures were continuously stirred at 250 rpm and room temperature in a 250-mL beaker. At pre-defined time-points (ranging from 0 to 60 min), 500 µL of sample were collected, centrifuged and filtered (200-nm pore size filtration membrane) before measurements. The concentration was evaluated by UV-Vis spectrophotometry (λ = 366 nm), using a Tecan Infinite M200 spectrometer.

Faster dissolution was obtained for the samples AMI,/GP-DCM-EtOH and AMI,/GP-DCM. In addition, 100% of amlodipine was dissolved after 30 minutes in the case of the AML/GP composites, independently of the solvent used, while only 80% of amlodipine was dissolved after 60 minutes in the case of crude amlodipine.

### Example 7 - Preparation of composites with improved dispersibility properties and controlled release

Composites of glucan particles and ibuprofen (IBU), as poorly-water soluble model low-molecular-weight compound, were prepared according to the procedure of Example 1, considering an IBU/GP mass ratio of 25 %. For that, 50-mL ibuprofen solution was prepared with concentration 5 mg/mL, using ethanol as organic solvent. Afterwards, 1.0 g of glucan particles was added to the solution and dispersed using an IKA^{®} T10 basic ultra-turrax for 5 minutes before spray drying. The sample was spray-dried using the Mini Spray Dryer B-290 equipped with the ultrasonic nozzle and operated in inert loop under N₂ atmosphere. The operating conditions used consisted of 120 °C inlet temperature, 5.0 mL/min feed rate, 246 L/h (20%) N₂ flow rate and 2.4 W power outlet at nozzle. The outlet temperature was 76 °C.

### Dispersion properties

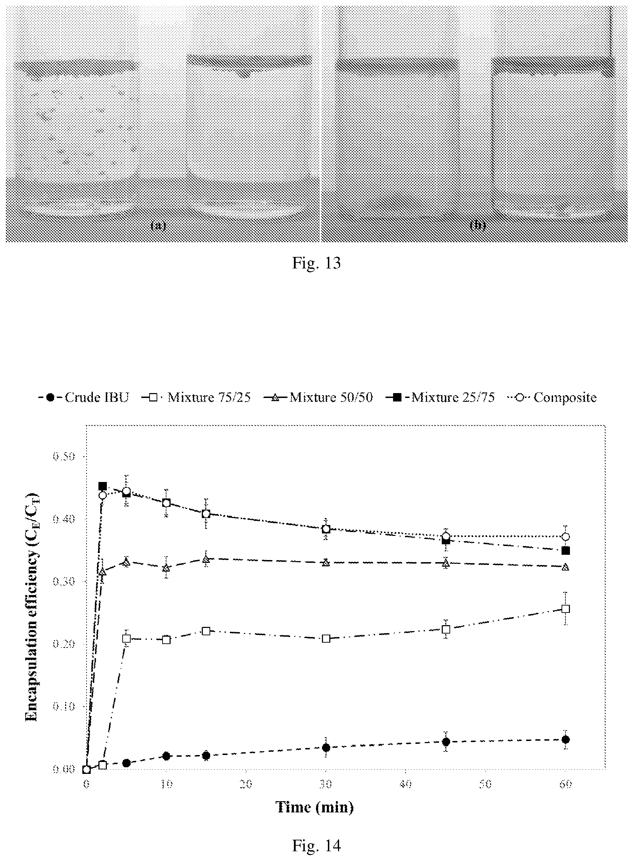

Dispersion properties of IBU/GP composites versus micronized crude ibuprofen (Fig. 13) were analyzed by observing the behavior of the samples in suspension. For that, 20.0 mg of each sample were weighted and added to 10.0 mL of 10 mM HCl (pH 2.0). The IBU/GP composites exhibit improved dispersion properties even without the use of a surfactant. Due to their good wettability, the dispersion of the composites was fast and spontaneous.

### Dissolution kinetics

Since ibuprofen is poorly soluble under acidic conditions, it can be expected that crystalline and amorphous forms of ibuprofen will show significantly different dissolution rates in acidic medium. Therefore, dissolution tests (Fig. 14) were performed for crude micronized ibuprofen (crystalline) and for the produced IBU/GP composite particles (amorphous), as well as for physical mixtures of the crude IBU and the composite particles, in powder form and using 10 mM HCl (pH 2.0) as dissolution medium. For that, 20.0 mg of ibuprofen (crude crystalline, GP composite or physical mixtures - see Table 3) were added to 200 mL of continuously stirred dissolution medium (250 rpm at room temperature) in a 250-mL beaker. At pre-defined time-points (ranging from 0 to 60 min), 500 µL of sample were collected, centrifuged and filtered (200-nm pore size filtration membrane), and the concentration was evaluated by high-performance liquid chromatography (HPLC) with UV detection (Agilent), coupled with C18 column (100 mm × 4.6 mm, 5 µm) and mobile phase consisting of 0.01 M ammonium phosphate buffer (pH 2.0) and acetonitrile (60%).

**Table 3:**

| Crystalline/amorphous ibuprofen proportion | Mass of crude ibuprofen (mg) | Mass of composite (mg) IBU/GP mass ratio = 25 % |
|---|---|---|
| 100 / 0 | 20.0 | 0.0 |
| 75 / 25 | 15.0 | 25.0 |
| 50 / 50 | 10.0 | 50.0 |
| 25 / 75 | 5.0 | 75.0 |
| 0 / 100 | 0.0 | 100.0 |

Progressively faster dissolution profiles were obtained with increasing mass fraction of encapsulated ibuprofen, until the solubility limit was reached. For the samples with the highest mass fraction of encapsulated ibuprofen (crystalline/amorphous ibuprofen proportion = 25/75 and 0/100), the fast release lead to supersaturation.

### Example 8 - Preparation of composites with improved powder flowability

Composites of glucan particles and atorvastatin (ATO), as poorly-water soluble model low-molecular-weight compound, were prepared according to the procedure of Example 1, considering an ATO/GP mass ratio of 25 %. For that, 50-mL atorvastatin solution was prepared with concentration 5 mg/mL, using ethanol as organic solvent. Afterwards, 1.0 g of glucan particles was added to the solution and dispersed using an IKA^{®} T10 basic ultra-turrax for 5 minutes before spray drying. The sample was spray-dried using the Mini Spray Dryer B-290 equipped with the ultrasonic nozzle and operated in inert loop under N₂ atmosphere. The operating conditions used consisted of 120 °C inlet temperature, 5.0 mL/min feed rate, 246 L/h (20%) N₂ flow rate and 2.4 W power outlet at nozzle. The outlet temperature was 76 °C.

Composites of glucan particles and atorvastatin (ATO) with ATO/GP mass ratio of 25 %, were also prepared by an alternative method, using rotary evaporator. For that, 200 mL of atorvastatin solution (1.25 mg/mL in ethanol) were added to 1.0 g of glucan particles in a round bottom flask. The obtained suspension was homogenized in an ultrasonication bath for 15 minutes, and the solvent was removed by evaporation using an IKA^{®} HB10 basic rotary evaporator. The operating conditions used consisted of 60 °C water-bath temperature and 175 RPM rotation speed. The pressure was slowly decreased from atmospheric pressure to 330 mBar. When most of the ethanol was removed, the pressure was decreased to 80-90 mBar, and the sample was dried at low pressure for 20 minutes. The obtained powder was collected from the round bottom flask and freeze-dried for 48 hours.

The samples are labelled as:
- ATO/GP-SD: For the composites prepared by spray drying.
- ATO/GP-RE: For the composites prepared by rotary evaporator.

Both composite samples (ATO/GP-SD and ATO/GP-RE) and crude atorvastatin were subjected to characterization.

### Powder rheology

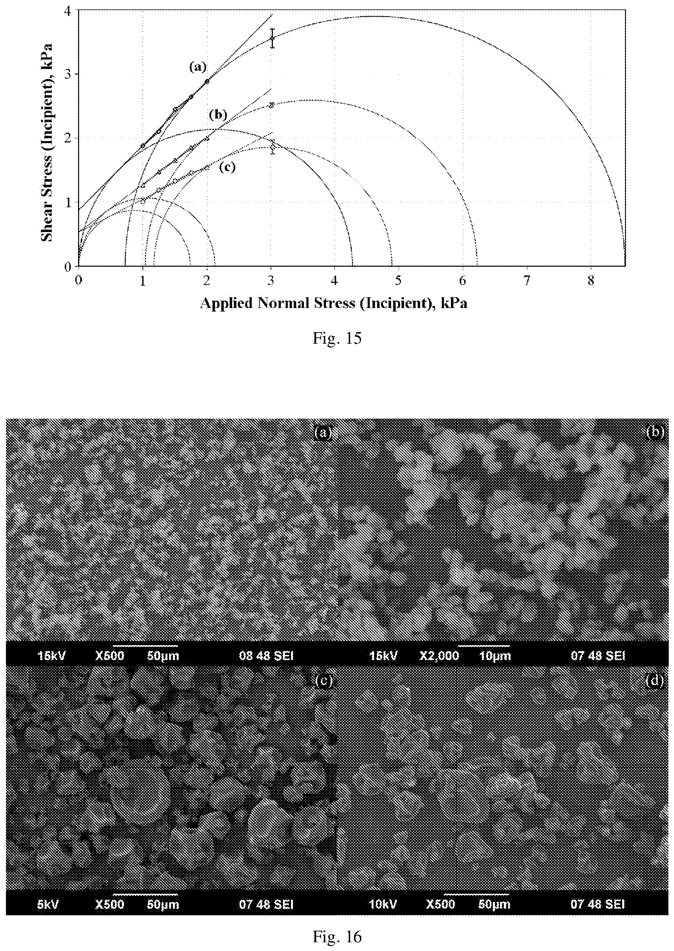

The moisture (water content) of the samples was firstly measure using a moisture analysis balance (simple test, 100 °C, 5 mg initial mass, infrared drying). The samples, ATO/GP-SD, ATO/GP-RE and crude ATO, contained 5 % wt., 3 % wt. and 2.5 % wt. of moisture respectively. Afterwards, the samples were exposed to laboratory humidity (21 °C and 28 % relative humidity), for 24 hours and the moisture content was measured again (9 % wt. for ATO/GP-SD, 9 % for ATO/GP-RE, and 5 % for ATO). The samples were then dried for 24 hours in the oven with very-slowly-moving fan at 30 °C. Finally, a shear test (Fig. 15) was performed on the samples a Powder Rheometer FT4 (pre-sheared at 3 kPa consolidation and initial mass of 0.6-0.7 g).

Crude atorvastatin exhibits the highest cohesion and internal friction, while the composites (ATO/GP-SD and ATO/GP-RE), both show improved flowability (Table 4). For both composite samples, cohesion is the same, but they differ in internal friction. Therefore, ATO/GP-SD and ATO/GP-RE composite samples will flow similarly in high-shear and compressive specific processes, but the spray-dried sample (ATO/GP-SD) has improved flowability than ATO/GP-RE in low-stress processes.

**Table 4:**

| Sample | Cohesion (kPa) | UYS* (kPa) | AIF* (°) |
|---|---|---|---|
| Crude ATO | 0.879 | 4.27 | 45.3 |
| ATO/GP-RE | 0.534 | 2.12 | 36.6 |
| ATO/GP-SD | 0.529 | 1.74 | 27.3 |

| | | | |
|---|---|---|---|
| *UYS: Unconfined Yield Strength; AIF: Angle of Internal Friction. | | | |

### Comparative Example 9 - Preparation of composites using polymeric matrices other than yeast glucan particles

Composites of glucan particles and atorvastatin (ATO), as poorly-water soluble model low-molecular-weight compound, were prepared according to the procedure of Example 1, considering an ATO/GP mass ratio of 10 %. For that, 50-mL atorvastatin solution was prepared with concentration 2 mg/mL, using ethanol as organic solvent. Afterwards, 1.0 g of glucan particles was added to the solution and dispersed using an IKA^{®} T10 basic ultra-turrax for 5 minutes before spray drying. The sample was spray-dried using the Mini Spray Dryer B-290 equipped with the ultrasonic nozzle and operated in inert loop under N₂ atmosphere. The operating conditions used consisted of 120 °C inlet temperature, 5.0 mL/min feed rate, 246 L/h (20%) N₂ flow rate and 2.4 W power outlet at nozzle. The outlet temperature was 76 °C.

For comparison, composites of hydrophilic polymers and atorvastatin with ATO/polymer mass ratio of 10 % were also prepared. The selected polymers were polyvinylpyrrolidone (PVP) and polyvinyl caprolactam-polyvinyl acetate-polyethylene glycol graft copolymer (Soluplus). These polymers are commonly used to produce amorphous solid dispersions. For the preparation of the composites, 50-mL atorvastatin solutions were prepared with concentration 2 mg/mL, using ethanol as organic solvent. Afterwards, 1.0 g of polymer was added to the solution and mixed until complete dissolution. Each sample was spray-dried using the same conditions as described above for ATO/GP. The composite with PVP is labelled as "ATO/PVP", and the composite with Soluplus is labelled as "ATO/SLP".

### Morphology of the composites

The morphology of the composites (Fig. 16) was evaluated by Scanning Electron Microscopy (SEM) using a Jeol JCM-5700 microscope. Before the SEM analysis, the samples were coated with a 5-nm gold layer using an Emitech K550X sputter coating equipment.

The ATO/GP composites present the typical ellipsoidal morphology with 2-4 µm particle size, exhibiting a wrinkled surface that can be attributed to the hydrolysis of the yeast outer cell wall and intercellular components, product of the alkaline and acid treatments. No evidence of atorvastatin outside of the glucan particles is observed. In the case of the composites with ATO/PVP and ATO/SLP, the particles present mushroom-like morphology, with much larger particle sizes, ranging between approximately 5 to 50 µm.

### Encapsulation efficiency

For the determination of the encapsulation efficiency, atorvastatin was extracted from the produced composites by adding 10.0 mg of the particles to 10.0 mL of methanol, in which atorvastatin is freely soluble. The dispersions were placed in an ultrasonication bath for 10 min to guarantee the complete extraction of the atorvastatin from the composites. Afterwards, the samples were centrifuged (5 min at 7000 rpm), and 500 µL of supernatant were collected. The concentration was evaluated by high-performance liquid chromatography (HPLC) with UV detection (Agilent), coupled with C18 column (100 mm × 4.6 mm, 5 µm) and mobile phase consisting of 0.01 M ammonium phosphate buffer (pH 2.0) and acetonitrile (60%).

The encapsulation efficiency of the composites was calculated as the experimental concentration of active compound (C_{E}), measured by HPLC, divided by the theoretical concentration (C_{T}) of atorvastatin in the composites. The highest encapsulation efficiency (C_{E}/C_{T}) was obtained for the ATO/GP composite followed by ATO/SLP sample and ATO/PVP, as shown in Table 5.

**Table 5:**

| Sample | Encapsulation efficiency [%] |
|---|---|
| ATO/GP | 92.40 ± 0.03 |
| ATO/SLP | 89.2 ± 0.01 |
| ATO/PVP | 75.60 ± 0.02 |

### Powder rheology

Shear tests were performed on the samples a Powder Rheometer FT4 (pre-sheared at 3 kPa consolidation and initial mass of 0.6-0.7 g). The tests were carried out per duplicate under laboratory conditions (21.2 ± 0.7 °C and 35.1 ± 2.0% relative humidity).

Crude atorvastatin exhibits the highest cohesion and internal friction (see Table 6). ATO/PVP also shows high cohesion but slightly lower than crude ATO. On the other hand, ATO/GP and ATO/SLP samples are the best flowable materials, belonging to the "easy flowing" materials category.

**Table 6:**

| Sample | Cohesion (kPa) | UYS* (kPa) | AIF* (°) | FF* | Category |
|---|---|---|---|---|---|
| Crude ATO | 0.81 ± 0.21 | 4.57 ± 1.07 | 51.24 ± 0.82 | 2.43 ± 0.48 | Cohesive |
| ATO/GP | 0.31 ± 0.01 | 1.14 ± 0.04 | 33.52 ± 0.09 | 4.33 ± 0.14 | Easy flowing |
| ATO/SLP | 0.30 ± 0.02 | 1.04 ± 0.05 | 29.60 ± 0.12 | 4.74 ± 0.22 | Easy flowing |
| ATO/PVP | 0.65 ± 0.04 | 2.10 ± 0.12 | 26.35 ± 0.11 | 2.65 ± 0.06 | Cohesive |

| | | | | | |
|---|---|---|---|---|---|
| *UYS: Unconfined Yield Strength; AIF: Angle of Internal Friction; FF: Flow Function. | | | | | |

### Comparative Example 10 - Preparation of yeast glucan particles spray dried from pure water, pure organic solvent and water/organic solvent mixtures

Given the hydrophilic nature of beta glucans, it is of interest to evaluate the effect of the use of water as a solvent or co-solvent in the preparation of spray-dried pure yeast glucan particles. Pure glucan particles were prepared according to the procedure of Example 1, using ethanol as organic solvent. For that, 1.0 g of glucan particles was added to 50 mL of ethanol and dispersed using an IKA^{®} T10 basic ultra-turrax for 5 minutes before spray drying. The sample was spray-dried using the Mini Spray Dryer B-290 equipped with the ultrasonic nozzle and operated in inert loop under N₂ atmosphere. The operating conditions used consisted of 120 °C inlet temperature, 5.0 mL/min feed rate, 246 L/h (20%) N₂ flow rate and 2.4 W power outlet at nozzle. The outlet temperature was from 60 to 70 °C.

Alternatively, for comparison, pure glucan particles were prepared using water and water/ethanol mixture (50/50) as solvents. For that, each sample was spray-dried using the Mini Spray Dryer B-290 equipped with the ultrasonic nozzle and operated under air atmosphere. The inlet temperature and power outlet at nozzle were adjusted adequately for each solvent. The operating conditions used consisted of 130-140 °C inlet temperature, 5.0 mL/min feed rate, 246 L/h (20%) air flow rate and 3.0 W power outlet at nozzle. The outlet temperature was from 60 to 70 °C. The samples are labelled according to the solvent used as GP-EtOH, GP-water, GP-EtOH/water respectively for ethanol, water and ethanol/water mixture.

### Morphology of the pure glucan particles

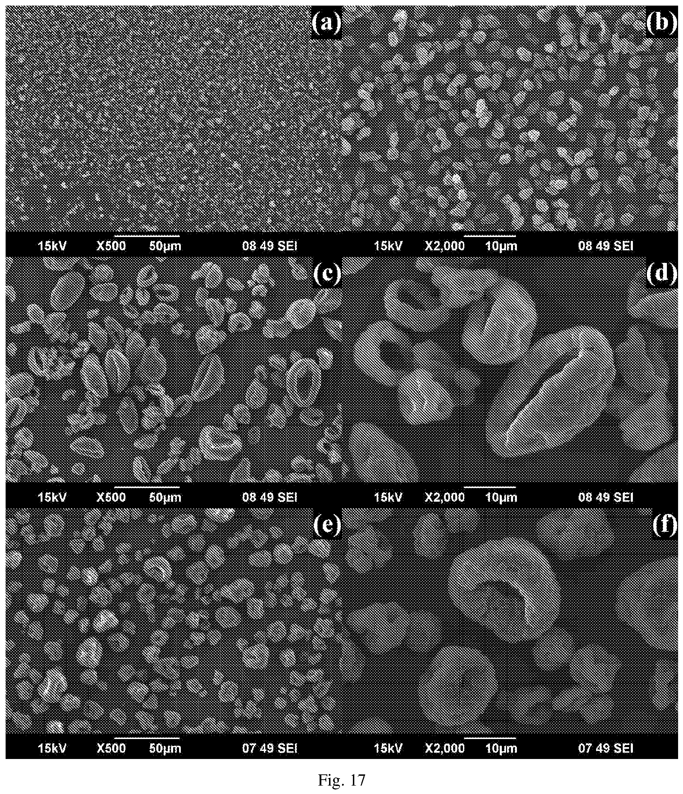

The morphology of the pure glucan particles (Fig. 17) was evaluated by Scanning Electron Microscopy (SEM) using a Jeol JCM-5700 microscope. Before the SEM analysis, the samples were coated with a 5-nm gold layer using an Emitech K550X sputter coating equipment.

The glucan particles spray dried from organic solvent (GP-EtOH) preserve the typical ellipsoidal morphology with 2-4 µm particle size, and wrinkled surface, the same morphology as was observed for GPs prepared in Example 1 and 2, whereas the samples prepared from water and water/ethanol mixture (GP-water and GP-EtOH/water) present mushroom-like morphology, and much larger particle sizes, ranging between approximately 5 to 50 µm.

### Particle size distributions

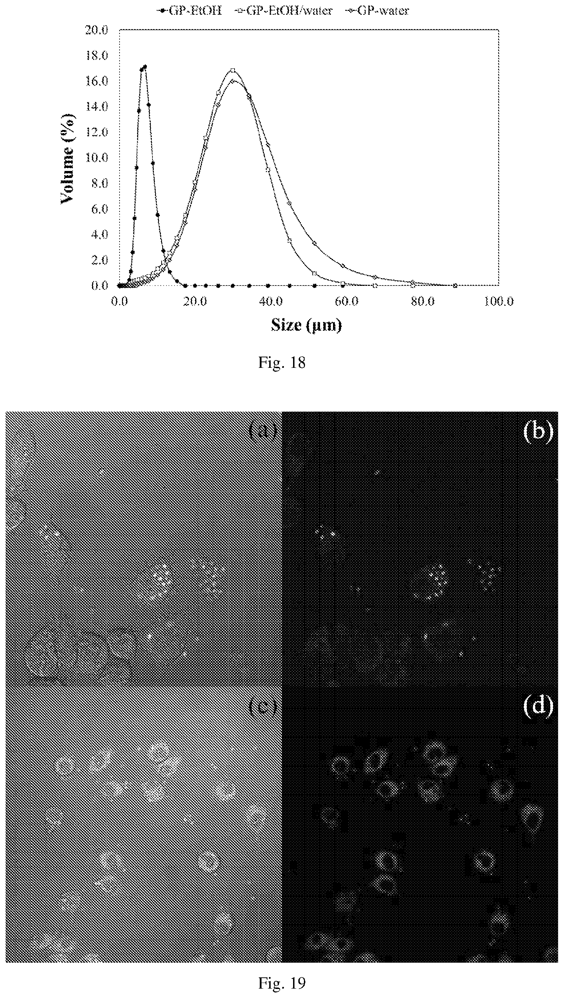

Particle size distributions of the samples (Fig. 18) were obtained by static light scattering using Horiba Partica LA 950/S2 equipment. Prior measurement, the samples were dispersed in distilled water at a concentration of 1.0 g/L and homogenized using an IKA^{®} T10 basic ultra-turrax for 1 minute. Mean size, D(v, 0.1), D(v, 0.5), and D(v, 0.9) are shown in Table 7.

**Table 7:**

| Sample | Mean Size (µm) | D(v, 0.1) (µm) | D(v, 0.5) (µm) | D(v,0.9) (µm) |
|---|---|---|---|---|
| GP-EtOH | 6.17396 ± 1.9706 | 3.92591 | 5.89407 | 8.78317 |
| GP-EtOH/water | 24.80679 ± 9.0110 | 12.81026 | 25.04354 | 36.24339 |
| GP-water | 27.94065 ± 10.2500 | 15.73663 | 27.02836 | 41.11664 |

In all cases, particle size distributions are monodispersed with sizes ranges in accordance to what was observed in the SEM images. The particle size of GPs has a fundamental influence on the phagocytosis by macrophages. It has been proven that particles in the size range of 0. 1-10 µm are the most biologically active in macrophage immune response. Given that human macrophages are about 21 µm in size, the engulfment of particles that are larger than themselves is limited and can potentially cause the death of the cells. Therefore, it is expected that macrophages can phagocytize small particles, such as GP-EtOH, more efficiently and in larger amounts than bigger particles, such as GP-EtOH/water and GP-water.

### In-vitro phagocytosis by macrophages

Phagocytosis by macrophages was evaluated for pure yeast glucan particles prepared using ethanol as solvent (GP-EtOH). First, a cell line J774A.1 (mouse macrophages) was cultivated using the culture method recommended by ATCC: The Global Bioresource Centre. The cells were cultivated by resuspending approximately 75 000 cells/well in 0.5 ml of FluoroBrite^{™} DMEM medium/well. Separately, the glucan particles were labelled using curcumin and Nile Red (GP/CC-EtOH and GP/NR-EtOH respectively). The labelled glucan particles were suspended in a concentration of 0.8 mg/ml of FluoroBrite^{™} DMEM medium and homogenized using an IKA^{®} T10 basic ultra-turrax for 1 minute. The suspensions of labelled glucan particles were added into the wells containing the macrophages in volumes of 3, 6 and 9 µL/well. Macrophages without labelled glucan particles were used as a control group. The cells were incubated at 37°C, 5% CO₂ and >93% relative humidity. The interaction of macrophages with labelled glucan particles was observed after 3, 5- and 24-hours using Olympus Fluoview FV1000 confocal system (405 nm and 550 nm excitation wavelength) and the scans were analyzed by Imaris (program for analysis of confocal scans).

The phagocytosis of few composites or dyed glucan particles was observed after 3 hours (Fig. 19). The macrophages show the highest phagocytosis activity after 5 hours. After 24 hours some macrophages saturated with microparticles swelled and died, but most of macrophages revealed phagocytosed the labelled glucan particles inside the cell body.

On the other hand, due to their large size, phagocytosis of GPs prepared from water and water/ethanol mixture (GP-water and GP-EtOH/water) by macrophages is expected to be limited and even cause macrophage's death.

## Claims

1. A method of production of a composite of yeast-derived beta-glucan particle with incorporated poorly-water-soluble low-molecular-weight compound, the poorly-water-soluble low-molecular-weight compound having solubility in 10 mM PBS of at most 30 mg/mL, measured at 37 °C and pH 7.4, and molecular mass of at most 5,000 Da, and weight ratio of the poorly-water-soluble low-molecular-weight compound to the yeast-derived beta-glucan particle is in the range of from 0.1·10⁻³ to 3, comprising the following steps:
i) the poorly-water-soluble low-molecular low-molecular-weight compound is dissolved in an organic solvent, selected from a group comprising ethanol, methanol, acetone, isopropanol, ethylacetate, dichloromethane, trichloromethane, chloroform, hexane, cyclohexane, heptane, toluene or mixtures thereof;
ii) yeast-derived beta glucan particles are added to the solution from step i) to form a suspension;
iii) the suspension obtained in step ii) is spray dried under inert atmosphere to form the composite of yeast-derived beta glucan particle with poorly-water-soluble low-molecular-weight compound incorporated in its amorphous form inside the yeast-derived glucan particles.

2. The method according to claim 1, wherein the concentration of the solution of the poorly-water-soluble low-molecular-weight compound in the organic solvent in step i) is up to and including 150 mg/ml.

3. The method according to claim 1 or 2, wherein the suspension in step ii) has concentration of from 50 mg to 4 g of beta glucan particles per 100 ml of solution from step i).

4. The method according to any one of claims 1 to 3, wherein the step iii) of spray drying is performed at volumetric gas-to-liquid flow ratio of from 50 to 10,000, and temperature in the range of from 30 to 350 °C.

5. The method according to any one of claims 1 to 4, wherein the beta glucan particles are obtained from *Saccharomyces cerevisiae.*

6. The method according to claim 5, wherein the beta-glucan particles are prepared by alkaline and acidic treatments of *Saccharomyces cerevisiae,* comprising the following steps:
a) natural or dried yeast is mixed with aqueous hydroxide, preferably in 1M NaOH or KOH, forming a suspension;
b) the suspension from step a) is homogenized and heated to at least 50 °C for at least 1 hour, preferably heated to 95 °C for 1 hour;
c) the suspension from step b) is centrifuged and the supernate is removed;
d) aqueous inorganic acid is added to the solid residue to adjust pH to about 4-5, and the suspension is heated to at least 50 °C for at least 2 hours;
e) the suspension from step d) is centrifuged and the supernate is removed;
f) the solid residue from step e) is washed with water and eventually water-miscible organic solvents, preferably selected from the group comprising isopropanol and acetone, and freeze-dried.

7. The method according to any one of the preceding claims 1 to 6, wherein the poorly-water-soluble low-molecular-weight compound is selected from a group comprising ibuprofen, curcumin, atorvastatin, diplacone, artemisinin, morusin, epigallocatechin gallate, resveratrol, acetylsalicylic acid, nilotinib, ellagic acid, acetyl-boswellic acid, and amlodipine.

8. A composite of yeast-derived beta-glucan particle with one or more incorporated poorly-water-soluble low-molecular-weight compounds, the poorly-water-soluble low-molecular-weight compound having solubility in 10 mM PBS of at most 30 mg/mL, measured at 37 °C and pH 7.4, and molecular mass of at most 5,000 Da, obtainable by the method according to any one of the preceding claims 1 to 7, wherein the weight ratio of the poorly-water-soluble low-molecular-weight compound to the yeast-derived beta-glucan particle is in the range of from 0.1·10⁻³ to 3, and wherein the poorly-water-soluble low-molecular-weight compound incorporated in the yeast-derived beta-glucan particle is in its amorphous form.

9. The composite according to claim 8, wherein the poorly-water-soluble low-molecular-weight compound is selected from the group comprising ibuprofen, curcumin, atorvastatin, diplacone, artemisinin, morusin, epigallocatechin gallate, resveratrol, acetylsalicylic acid, nilotinib, ellagic acid, acetyl-boswellic acid, and amlodipine.

10. A pharmaceutical composition for gastrointestinal administration, **characterized in that** it comprises the composite according to claim 8 or 9 as a carrier of the poorly-water-soluble low-molecular-weight compound, wherein the poorly-water soluble low-molecular-weight compound incorporated in the beta-glucan particle is a medicament, and at least one pharmaceutically acceptable carrier, selected from the group comprising fillers, stabilizers, excipients, binders, disintegrants.

11. The pharmaceutical composition according to claim 10, **characterized in that** it further comprises a poorly-water-soluble low-molecular-weight medicament in crystalline form, not encapsulated in glucan particles, wherein the poorly-water-soluble medicament in crystalline form has solubility in 10 mM PBS of at most 30 mg/mL, measured at 37 °C and pH 7.4, and molecular mass of at most 5,000 Da.

12. The pharmaceutical composition according to claim 11, wherein the poorly-water-soluble low-molecular-weight medicament in crystalline form is selected from the group comprising ibuprofen, curcumin, atorvastatin, diplacone, artemisinin, morusin, epigallocatechin gallate, resveratrol, acetylsalicylic acid, nilotinib, ellagic acid, acetyl-boswellic acid and amlodipine.

13. The composite according to claim 8 or 9 and/or the pharmaceutical composition according to any one of claims 10 to 12 for use as a carrier of the poorly-water-soluble low-molecular-weight compound in medicine.

14. The pharmaceutical composition according to any one of claims 11 to 12 for use as a controlled release medicament.

15. Use of the composite according to claim 8 as a food supplement.

## Patentansprüche

1. Verfahren zur Herstellung eines Verbundstoffs aus Hefe-abgeleitetem Beta-Glucan-Partikel mit eingebauter schlecht wasserlöslicher niedermolekularer Verbindung, wobei die schlecht wasserlösliche niedermolekulare Verbindung eine Löslichkeit in 10 mM PBS von höchstens 30 mg/ml, gemessen bei 37 °C und pH 7,4, und eine Molekularmasse von höchstens 5.000 Da aufweist und das Gewichtsverhältnis der schlecht wasserlöslichen niedermolekularen Verbindung zu dem Hefe-abgeleiteten Beta-Glucan-Partikel im Bereich von 0,1·10⁻³ bis 3 liegt, das Verfahren umfassend die folgenden Schritte:
i) die schlecht wasserlösliche niedermolekulare Verbindung wird in einem organischen Lösungsmittel gelöst, ausgewählt aus einer Gruppe umfassend Ethanol, Methanol, Aceton, Isopropanol, Ethylacetat, Dichlormethan, Trichlormethan, Chloroform, Hexan, Cyclohexan, Heptan, Toluol oder Mischungen davon;
ii) Hefe-abgeleitete Beta-Glucan-Partikel werden der Lösung aus Schritt i) hinzugefügt, um eine Suspension zu bilden;
iii) die in Schritt ii) erhaltene Suspension wird unter inerter Atmosphäre sprühgetrocknet, um den Verbundstoff aus Hefe-abgeleiteten Beta-Glucan-Partikeln mit schlecht wasserlöslicher niedermolekularer Verbindung, die in ihrer amorphen Form in die Hefe-abgeleiteten Glucan-Partikel eingearbeitet ist, zu bilden.

2. Verfahren nach Anspruch 1, wobei die Konzentration der Lösung der schlecht wasserlöslichen niedermolekularen Verbindung im organischen Lösungsmittel in Schritt i) bis zu einschließlich 150 mg/ml beträgt.

3. Verfahren nach Anspruch 1 oder 2, wobei die Suspension in Schritt ii) eine Konzentration von 50 mg bis 4 g Beta-Glucan-Partikeln pro 100 ml Lösung aus Schritt i) aufweist.

4. Verfahren nach einem der Ansprüche 1 bis 3, wobei der Schritt iii) des Sprühtrocknens bei einem volumetrischen Gas-Flüssigkeits-Flussverhältnis von 50 bis 10.000 und einer Temperatur im Bereich von 30 bis 350 °C durchgeführt wird.

5. Verfahren nach einem der Ansprüche 1 bis 4, wobei die Beta-Glucan-Partikel aus *Saccharomyces cerevisiae* gewonnen werden.

6. Verfahren nach Anspruch 5, wobei die Beta-Glucan-Partikel durch alkalische und saure Behandlungen von *Saccharomyces cerevisiae* hergestellt werden, die Behandlung umfassend die folgenden Schritte:
a) natürliche oder getrocknete Hefe wird mit wässrigem Hydroxid, vorzugsweise 1 M NaOH oder KOH, gemischt, wodurch eine Suspension entsteht;
b) die Suspension aus Schritt a) wird homogenisiert und mindestens 1 Stunde lang auf mindestens 50 °C, vorzugsweise 1 Stunde lang auf 95 °C, erhitzt;
c) die Suspension aus Schritt b) wird zentrifugiert und der Überstand wird entfernt;
d) dem festen Rückstand wird wässrige anorganische Säure zugesetzt, um den pH-Wert auf etwa 4-5 einzustellen, und die Suspension wird mindestens 2 Stunden lang auf mindestens 50 °C erhitzt;
e) die Suspension aus Schritt d) wird zentrifugiert und der Überstand wird entfernt;
f) der feste Rückstand aus Schritt e) wird mit Wasser und gegebenenfalls wassermischbaren organischen Lösungsmitteln, vorzugsweise ausgewählt aus der Gruppe umfassend Isopropanol und Aceton, gewaschen und gefriergetrocknet.

7. Verfahren nach einem der vorhergehenden Ansprüche 1 bis 6, wobei die schlecht wasserlösliche niedermolekulare Verbindung ausgewählt ist aus einer Gruppe umfassend Ibuprofen, Curcumin, Atorvastatin, Diplacon, Artemisinin, Morusin, Epigallocatechin-Gallat, Resveratrol, Acetylsalicylsäure, Nilotinib, Ellagsäure, Acetylboswelliasäure und Amlodipin.

8. Einer Verbundstoff aus Hefe-abgeleitetem Beta-Glucan-Partikel mit einer oder mehreren eingearbeiteten schlecht wasserlöslichen niedermolekularen Verbindungen, wobei die schlecht wasserlösliche niedermolekulare Verbindung eine Löslichkeit in 10 mM PBS von höchstens 30 mg/ml, gemessen bei 37 °C und pH 7,4, und eine Molekularmasse von höchstens 5.000 Da aufweist, erhältlich durch das Verfahren gemäß einem der vorhergehenden Ansprüche 1 bis 7, wobei das Gewichtsverhältnis der schlecht wasserlöslichen niedermolekularen Verbindung zu dem Hefe-abgeleiteten Beta-Glucan-Partikel im Bereich von 0,1·10⁻³ bis 3 liegt und wobei die schlecht wasserlösliche niedermolekulare Verbindung, die in das Hefe-abgeleitete Beta-Glucan-Partikel eingearbeitet ist, in ihrer amorphen Form vorliegt.

9. Der Verbundstoff nach Anspruch 8, wobei die schlecht wasserlösliche niedermolekulare Verbindung ausgewählt ist aus der Gruppe umfassend Ibuprofen, Curcumin, Atorvastatin, Diplacon, Artemisinin, Morusin, Epigallocatechin-Gallat, Resveratrol, Acetylsalicylsäure, Nilotinib, Ellagsäure, Acetylboswelliasäure und Amlodipin.

10. Eine pharmazeutische Zusammensetzung zur gastrointestinalen Verabreichung, **dadurch gekennzeichnet, dass** sie den Verbundstoff nach Anspruch 8 oder 9 als Träger der schlecht wasserlöslichen niedermolekularen Verbindung umfasst, wobei die schlecht wasserlösliche niedermolekulare Verbindung, die in das Beta-Glucan-Partikel eingearbeitet ist, ein Medikament ist, und mindestens einen pharmazeutisch akzeptablen Träger, ausgewählt aus der Gruppe, die Füllstoffe, Stabilisatoren, Hilfsstoffe, Bindemittel, Sprengmittel umfasst.

11. Pharmazeutische Zusammensetzung nach Anspruch 10, **dadurch gekennzeichnet, dass** sie zusätzlich ein schlecht wasserlösliches niedermolekulares Arzneimittel in kristalliner Form, das nicht in Glucanpartikel eingekapselt ist, umfasst, wobei das schlecht wasserlösliche Arzneimittel in kristalliner Form hat eine Löslichkeit in 10 mM PBS von höchstens 30 mg/ml, gemessen bei 37 °C und pH 7,4, und eine Molekularmasse von höchstens 5.000 Da.

12. Die pharmazeutische Zusammensetzung nach Anspruch 11, wobei das schlecht wasserlösliche niedermolekulare Medikament in kristalliner Form ausgewählt ist aus der Gruppe umfassend Ibuprofen, Curcumin, Atorvastatin, Diplacon, Artemisinin, Morusin, Epigallocatechin-Gallat, Resveratrol, Acetylsalicylsäure, Nilotinib, Ellagsäure, Acetyl-Boswelliasäure und Amlodipin.

13. Der Verbundstoff nach Anspruch 8 oder 9 und/oder die pharmazeutische Zusammensetzung nach einem der Ansprüche 10 bis 12 zur Verwendung als Träger der schlecht wasserlöslichen niedermolekularen Verbindung in der Medizin.

14. Die pharmazeutische Zusammensetzung nach einem der Ansprüche 11 bis 12 zur Verwendung als Arzneimittel mit kontrollierter Freisetzung.

15. Verwendung des Verbundstoffs nach Anspruch 8 als Nahrungsergänzungsmittel.

## Revendications

1. Un procédé de production d'un composite de particules de bêta-glucane dérivées de levure avec un composé de bas poids moléculaire peu soluble dans l'eau incorporé, le composé de bas poids moléculaire peu soluble dans l'eau ayant une solubilité dans du PBS 10 mM au maximum de 30 mg/mL, mesurée à 37 °C et pH 7,4, et une masse moléculaire au maximum de 5 000 Da, et le rapport pondéral du composé de bas poids moléculaire peu soluble dans l'eau à la particule de bêta-glucane dérivée de levure est dans la plage de 0,1·10⁻³ à 3, comprenant les étapes suivantes :
i) le composé de bas poids moléculaire peu soluble dans l'eau est dissous dans un solvant organique, choisi dans un groupe comprenant l'éthanol, le méthanol, l'acétone, l'isopropanol, l'acétate d'éthyle, le dichlorométhane, le trichlorométhane, le chloroforme, l'hexane, le cyclohexane, l'heptane, le toluène ou leurs mélanges ;
ii) des particules de bêta-glucane dérivées de levure sont ajoutées à la solution de l'étape i) pour former une suspension ;
iii) la suspension obtenue à l'étape ii) est séchée par atomisation sous atmosphère inerte pour former le composite de particules de bêta-glucane dérivées de levure avec un composé de bas poids moléculaire peu soluble dans l'eau incorporé sous sa forme amorphe à l'intérieur des particules de bêta-glucane dérivées de levure.

2. Le procédé selon la revendication 1, dans lequel la concentration de la solution du composé de bas poids moléculaire peu soluble dans l'eau dans le solvant organique de l'étape i) est jusqu'à 150 mg/ml inclus.

3. Le procédé selon la revendication 1 ou 2, dans lequel la suspension de l'étape ii) a une concentration de 50 mg à 4 g de particules de bêta-glucane pour 100 ml de solution de l'étape i).

4. Le procédé selon l'une quelconque des revendications 1 à 3, dans lequel l'étape iii) de séchage par atomisation est réalisée à un rapport volumétrique gaz/liquide de 50 à 10 000 et à une température dans la plage de 30 à 350 °C.

5. Le procédé selon l'une quelconque des revendications 1 à 4, dans lequel les particules de bêta-glucane sont obtenues à partir de *Saccharomyces cerevisiae.*

6. Le procédé selon la revendication 5, dans lequel les particules de bêta-glucane sont préparées par des traitements alcalins et acides de *Saccharomyces cerevisiae,* comprenant les étapes suivantes :
a) la levure naturelle ou séchée est mélangée avec de l'hydroxyde aqueux, de préférence dans 1 M de NaOH ou de KOH, pour former une suspension ;
b) la suspension de l'étape a) est homogénéisée et chauffée à au moins 50 °C pendant au moins 1 heure, de préférence chauffée à 95 °C pendant 1 heure ;
c) la suspension de l'étape b) est centrifugée et le surnageant est éliminé ;
d) l'acide inorganique aqueux est ajouté au résidu solide pour ajuster le pH à environ 4-5, et la suspension est chauffée à au moins 50 °C pendant au moins 2 heures ;
e) la suspension de l'étape d) est centrifugée et le surnageant est éliminé ;
f) le résidu solide de l'étape e) est lavé avec de l'eau et éventuellement des solvants organiques miscibles à l'eau, de préférence choisis dans le groupe comprenant l'isopropanol et l'acétone, et lyophilisé.

7. Le procédé selon l'une quelconque des revendications 1 à 6 précédentes, dans lequel le composé de bas poids moléculaire peu soluble dans l'eau est choisi dans un groupe comprenant l'ibuprofène, la curcumine, l'atorvastatine, la diplacone, l'artémisinine, la morusine, le gallate d'épigallocatéchine, le resvératrol, l'acide acétylsalicylique, le nilotinib, l'acide ellagique, l'acide acétyl-boswellique et l'amlodipine.

8. Un composite de particules de bêta-glucane dérivées de levure avec un ou plusieurs composés de bas poids moléculaire peu solubles dans l'eau incorporés, le composé de bas poids moléculaire peu soluble dans l'eau ayant une solubilité dans du PBS 10 mM au maximum de 30 mg/mL, mesurée à 37 °C et pH 7,4, et une masse moléculaire au maximum de 5 000 Da, pouvant être obtenu par le procédé selon l'une quelconque des revendications 1 à 7 précédentes, dans lequel le rapport pondéral du composé de bas poids moléculaire peu soluble dans l'eau à la particule de bêta-glucane dérivée de levure est dans la plage de 0,1·10⁻³ à 3, et dans lequel le composé de bas poids moléculaire peu soluble dans l'eau incorporé dans la particule de bêta-glucane dérivée de levure est sous sa forme amorphe.

9. Le composite selon la revendication 8, dans lequel le composé de bas poids moléculaire peu soluble dans l'eau est choisi dans le groupe comprenant l'ibuprofène, la curcumine, l'atorvastatine, la diplacone, l'artémisinine, la morusine, le gallate d'épigallocatéchine, le resvératrol, l'acide acétylsalicylique, le nilotinib, l'acide ellagique, l'acide acétyl-boswellique et l'amlodipine.

10. Une composition pharmaceutique pour administration gastro-intestinale, **caractérisée en ce qu'**elle comprend le composite selon la revendication 8 ou 9 comme support du composé de bas poids moléculaire peu soluble dans l'eau, dans lequel le composé de bas poids moléculaire peu soluble dans l'eau incorporé dans la particule de bêta-glucane est un médicament, et au moins un support pharmaceutiquement acceptable, choisi dans le groupe comprenant des charges, des stabilisants, des excipients, des liants, des désintégrants.

11. La composition pharmaceutique selon la revendication 10, **caractérisée en ce qu'**elle comprend en outre un médicament de bas poids moléculaire peu soluble dans l'eau sous forme cristalline, non encapsulé dans des particules de glucane, dans laquelle le médicament peu soluble dans l'eau sous forme cristalline a une solubilité dans du PBS 10 mM au maximum de 30 mg/mL, mesurée à 37 °C et pH 7,4, et une masse moléculaire au maximum de 5 000 Da.

12. La composition pharmaceutique selon la revendication 11, dans laquelle le médicament de bas poids moléculaire peu soluble dans l'eau sous forme cristalline est choisi dans le groupe comprenant l'ibuprofène, la curcumine, l'atorvastatine, la diplacone, l'artémisinine, la morusine, le gallate d'épigallocatéchine, le resvératrol, l'acide acétylsalicylique, le nilotinib, l'acide ellagique, l'acide acétyl-boswellique et l'amlodipine.

13. Le composite selon la revendication 8 ou 9 et/ou la composition pharmaceutique selon l'une quelconque des revendications 10 à 12 pour utilisation comme support du composé de bas poids moléculaire peu soluble dans l'eau en médecine.

14. La composition pharmaceutique selon l'une quelconque des revendications 11 à 12 pour utilisation comme médicament à libération contrôlée.

15. Utilisation du composite selon la revendication 8 comme complément alimentaire.
